(19) Europäisches Patentamt
European Patent Office
Office européen des brevets

(11) EP 4 527 917 A1

(12) **EUROPEAN PATENT APPLICATION**
published in accordance with Art. 153(4) EPC

(43) Date of publication:
26.03.2025 Bulletin 2025/13

(51) International Patent Classification (IPC):
*C12M 3/00* (2006.01)   *C12N 5/071* (2010.01)
*C12M 1/00* (2006.01)

(21) Application number: 23807700.2

(52) Cooperative Patent Classification (CPC):
C12M 1/00; C12M 3/00; C12N 5/06

(22) Date of filing: 18.05.2023

(86) International application number:
PCT/JP2023/018621

(87) International publication number:
WO 2023/224099 (23.11.2023 Gazette 2023/47)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 20.05.2022 JP 2022082939

(71) Applicants:
• Public University Corporation
Nagoya City University
Nagoya-shi, Aichi 467-8601 (JP)
• Shinko Chemical Co., Ltd.
Kanazawa-shi
Ishikawa 920-0346 (JP)

(72) Inventors:
• MATSUNAGA, Tamihide
Nagoya-shi, Aichi 467-8603 (JP)
• IWAO, Takahiro
Nagoya-shi, Aichi 467-8603 (JP)
• SAKAI, Yoko
Nagoya-shi, Aichi 467-8603 (JP)
• OGAWA, Isamu
Nagoya-shi, Aichi 467-8603 (JP)
• YAMADA, Hideki
Kanazawa-shi, Ishikawa 920-0346 (JP)
• DOI, Atsushi
Kanazawa-shi, Ishikawa 920-0346 (JP)

(74) Representative: HGF
HGF Limited
1 City Walk
Leeds LS11 9DX (GB)

(54) **CELL CULTURE SUBSTRATE AND CELL CULTURE METHOD**

(57) A cell culture substrate according to the present disclosure is a cell culture substrate including at least one well including a side surface and a bottom surface, in which the side surface includes a first side portion defining a first space and a second side portion defining a second space communicating with the first space, and in plan view, an upper end portion of the first side portion is formed in a C shape having a central angle of 240 degrees or more, the second space extends from an opening portion of the upper end portion in a first direction away from the first space in the plan view, and the second side portion includes a facing surface facing the opening portion in the first direction, the facing surface being disposed at a position where a maximum distance between the opening portion and the facing surface in the first direction is smaller than a diameter of the C shape of the upper end portion in plan view.

Fig. 2A

## Description

TECHNICAL FIELD

The present disclosure relates to a cell culture substrate including a well for culturing a cell, and a cell culturing method using the cell culture substrate.

BACKGROUND ART

A cell culture test is performed using, for example, a resin substrate called a well plate (or a companion plate) provided with a plurality of wells. In each of the wells, a cell culture container called an insert is disposed, and cells are cultured in the insert. Cells grow by taking nutrients from a culture medium contained within the well and within the insert. Herein, a substrate provided with at least one well, such as a well plate, is referred to as a "cell culture substrate".

The culture medium in the well and the culture medium in the insert are discharged from the well by a pipette, an aspirator, or the like and replaced with a new culture medium as necessary. Discharge of the old culture medium in the well is usually performed with the insert removed from the well. This is because, in a state where the insert is disposed in the well, a gap between an inner surface of the well and the insert is narrow, and it is difficult to insert a distal end portion of a pipette or the like into the well without contacting the insert. However, it takes a lot of effort to remove the insert from each well each time the culture medium is replaced.

For example, Patent Document 1 discloses a cell culture device capable of discharging a culture medium from a well without removing an insert from the well.

PRIOR ART DOCUMENT

PATENT DOCUMENT

Patent Document 1: WO 2018/105078 A

SUMMARY OF THE INVENTION

PROBLEMS TO BE SOLVED BY THE INVENTION

The cell culture device disclosed in Patent Document 1 is configured to supply and discharge the culture medium through an insertion tube inserted inside the well and outside the insert. According to the configuration of Patent Document 1, the culture medium can be replaced in a state where the insert is disposed in the well.

However, in the configuration of Patent Document 1, there is room for improvement from the viewpoint of more stably holding the insert in the well. If the insert is not held stably, the insert may wobble or tilt during a replacement operation of the culture medium in the well. This may affect the cells within the insert. Furthermore, if the insert is not held stably, it may be difficult to discharge the culture medium within the insert. In particular, it is considered difficult to automatically (using a robot or the like) discharge the culture medium from the inside of the insert.

An object of the present disclosure is to solve the above problems, and to provide a cell culture substrate capable of replacing a culture medium while suppressing an influence on cultured cells.

SOLUTIONS TO THE PROBLEMS

In order to achieve the above object, the present disclosure is configured as follows.

According to a cell culture substrate according to one aspect of the present disclosure,

a cell culture substrate includes at least one well including a side surface and a bottom surface, the side surface includes a first side portion defining a first space and a second side portion defining a second space communicating with the first space, and

in plan view, an upper end portion of the first side portion is formed in a C shape having a central angle of 240 degrees or more,

the second space extends from an opening portion of the upper end portion in a first direction away from the first space in plan view, and

the second side portion includes a facing surface facing the opening portion in the first direction, the facing surface being disposed at a position where a maximum distance between the opening portion and the facing surface in the first direction is smaller than a diameter of the C shape of the upper end portion in plan view.

EFFECTS OF THE INVENTION

According to the present disclosure, it is possible to provide a cell culture substrate capable of replacing a culture medium while suppressing an influence on cultured cells.

BRIEF DESCRIPTION OF THE DRAWINGS

[Fig. 1] Fig. 1 is a perspective view of a well in a cell culture substrate according to an embodiment of the present disclosure.
[Fig. 2A] Fig. 2A is a plan view of the well of Fig. 1.
[Fig. 2B] Fig. 2B is a cross-sectional view taken along line A1-A1 of Fig. 1.
[Fig. 2C] Fig. 2C is a cross-sectional view taken along line B1-B1 in Fig. 1.
[Fig. 2D] Fig. 2D is a cross-sectional view taken along line B2-B2 in Fig. 1.
[Fig. 3A] Fig. 3A is a plan view exemplifying variations in shape and arrangement of grooves.

[Fig. 3B] Fig. 3B is a plan view exemplifying variations in shape and arrangement of grooves.

[Fig. 3C] Fig. 3C is a plan view exemplifying variations in shape and arrangement of grooves.

[Fig. 3D] Fig. 3D is a plan view exemplifying variations in shape and arrangement of grooves.

[Fig. 4] Fig. 4 is a perspective view exemplifying an insert.

[Fig. 5] Fig. 5 is a perspective view for explaining a support structure provided in the well of Fig. 1.

[Fig. 6A] Fig. 6A is a plan view illustrating an arrangement relationship between an anti-rotation rib of the well and a side wall of the insert illustrated in Fig. 4.

[Fig. 6B] Fig. 6B is a plan view illustrating a state in which another insert is supported in the well.

[Fig. 7A] Fig. 7A is a step cross-sectional view for explaining a cell culture method.

[Fig. 7B] Fig. 7B is a step cross-sectional view for explaining a cell culture method.

[Fig. 8A] Fig. 8A is a step cross-sectional view for explaining a cell culture method.

[Fig. 8B] Fig. 8B is a step cross-sectional view for explaining a cell culture method.

[Fig. 9A] Fig. 9A is a step cross-sectional view for explaining a cell culture method.

[Fig. 9B] Fig. 9B is a step cross-sectional view for explaining a cell culture method.

[Fig. 10A] Fig. 10A is a step cross-sectional view for explaining a cell culture method.

[Fig. 10B] Fig. 10B is a step cross-sectional view for explaining a cell culture method.

[Fig. 11A] Fig. 11A is a step cross-sectional view for explaining a cell culture method.

[Fig. 11B] Fig. 11B is a step cross-sectional view for explaining a cell culture method.

[Fig. 12A] Fig. 12A is a step cross-sectional view for explaining a cell culture method.

[Fig. 12B] Fig. 12B is a step cross-sectional view for explaining a cell culture method.

[Fig. 13] Fig. 13 is a perspective view illustrating an example of a cell culture substrate.

[Fig. 14] Fig. 14 is a plan view of the cell culture substrate illustrated in Fig. 13.

[Fig. 15] Fig. 15 is a perspective view illustrating a state in which the cell culture substrate is tilted in an X direction.

[Fig. 16] Fig. 16 is a plan view illustrating a modification of the cell culture substrate of Fig. 13.

[Fig. 17] Fig. 17 is a diagram illustrating measurement results of TEER values in a cell culture experiment 1.

[Fig. 18A] Fig. 18A is a diagram illustrating mRNA expression analysis results in the cell culture experiment 1.

[Fig. 18B] Fig. 18B is a diagram illustrating mRNA expression analysis results in the cell culture experiment 1.

[Fig. 18C] Fig. 18C is a diagram illustrating mRNA expression analysis results in the cell culture experiment 1.

[Fig. 18D] Fig. 18D is a diagram illustrating mRNA expression analysis results in the cell culture experiment 1.

[Fig. 18E] Fig. 18E is a diagram illustrating mRNA expression analysis results in the cell culture experiment 1.

[Fig. 19A] Fig. 19A is a diagram illustrating measurement results of drug metabolizing enzyme activity in the cell culture experiment 1.

[Fig. 19B] Fig. 19B is a diagram illustrating measurement results of drug metabolizing enzyme activity in the cell culture experiment 1.

[Fig. 19C] Fig. 19C is a diagram illustrating measurement results of drug metabolizing enzyme activity in the cell culture experiment 1.

[Fig. 19D] Fig. 19D is a diagram illustrating measurement results of drug metabolizing enzyme activity in the cell culture experiment 1.

[Fig. 20A] Fig. 20A is a phase contrast microscopic image of a cell surface day 6 after seeding in a cell culture experiment 2.

[Fig. 20B] Fig. 20B is a phase contrast microscopic image of a cell surface day 6 after seeding in a cell culture experiment 2.

[Fig. 20C] Fig. 20C is a phase contrast microscopic image of a cell surface day 6 after seeding in a cell culture experiment 2.

[Fig. 21A] Fig. 21A is a phase contrast microscopic image of a cell surface day 7 after seeding in the cell culture experiment 2.

[Fig. 21B] Fig. 21B is a phase contrast microscopic image of a cell surface day 7 after seeding in the cell culture experiment 2.

[Fig. 21C] Fig. 21C is a phase contrast microscopic image of a cell surface day 7 after seeding in the cell culture experiment 2.

[Fig. 22A] Fig. 22A is a phase contrast microscopic image of a cell surface day 8 after seeding in the cell culture experiment 2.

[Fig. 22B] Fig. 22B is a phase contrast microscopic image of a cell surface day 8 after seeding in the cell culture experiment 2.

[Fig. 22C] Fig. 22C is a phase contrast microscopic image of a cell surface day 8 after seeding in the cell culture experiment 2.

[Fig. 23A] Fig. 23A is a phase contrast microscopic image of a cell surface day 9 after seeding in the cell culture experiment 2.

[Fig. 23B] Fig. 23B is a phase contrast microscopic image of a cell surface day 9 after seeding in the cell culture experiment 2.

[Fig. 23C] Fig. 23C is a phase contrast microscopic image of a cell surface day 9 after seeding in the cell culture experiment 2.

[Fig. 24A] Fig. 24A is a phase contrast microscopic image of a cell surface day 10 after seeding in the cell culture experiment 2.

[Fig. 24B] Fig. 24B is a phase contrast microscopic image of a cell surface day 10 after seeding in the cell culture experiment 2.

[Fig. 24C] Fig. 24C is a phase contrast microscopic image of a cell surface day 10 after seeding in the cell culture experiment 2.

[Fig. 25] Fig. 25 is a diagram illustrating measurement results of TEER values in the cell culture experiment 2.

[Fig. 26A] Fig. 26A is a diagram illustrating mRNA expression analysis results in the cell culture experiment 2.

[Fig. 26B] Fig. 26B is a diagram illustrating mRNA expression analysis results in the cell culture experiment 2.

[Fig. 26C] Fig. 26C is a diagram illustrating mRNA expression analysis results in the cell culture experiment 2.

[Fig. 26D] Fig. 26D is a diagram illustrating mRNA expression analysis results in the cell culture experiment 2.

[Fig. 26E] Fig. 26E is a diagram illustrating mRNA expression analysis results in the cell culture experiment 2.

[Fig. 26F] Fig. 26F is a diagram illustrating mRNA expression analysis results in the cell culture experiment 2.

[Fig. 26G] Fig. 26G is a diagram illustrating mRNA expression analysis results in the cell culture experiment 2.

[Fig. 26H] Fig. 26H is a diagram illustrating mRNA expression analysis results in the cell culture experiment 2.

[Fig. 26I] Fig. 26I is a diagram illustrating mRNA expression analysis results in the cell culture experiment 2.

[Fig. 26J] Fig. 26J is a diagram illustrating mRNA expression analysis results in the cell culture experiment 2.

## DETAILED DESCRIPTION

(Knowledge underlying the present disclosure)

The present inventors have intensively studied the configuration of a cell culture substrate capable of replacing a culture medium while suppressing an influence on cultured cells, and as a result, have obtained the following findings.

In order to perform the culture medium replacement work without removing an insert from a well, it is conceivable to secure a space for inserting a tube such as a pipette in the well separately from a space in which the insert is disposed.

In a conventional well plate, a well having a hollow cylindrical shape is provided. The insert is disposed, for example, substantially at a center of the circular well in plan view. When a space for inserting a tube such as a pipette is provided inside the well having such a shape, it becomes difficult to stably hold the insert. For example, in Patent Document 1, in order to secure the space for inserting the tube into the well, an insert having a shape in which a part of a cylindrical shape is cut out is used. For this reason, the balance of the insert is poor, and when the culture medium in the well is replaced, the insert may wobble due to the flow of the culture medium or the like. Moreover, when the well is tilted to efficiently discharge the culture medium, there is a possibility that the insert may deviate from a predetermined position or tilt and hit an inner surface of the well. Then, the cells in the insert may be damaged, such as the cells being peeled off from a bottom surface of the insert. Furthermore, since the balance of the insert is poor, it is also difficult to replace the culture medium in the insert.

Therefore, as a result of repeated studies, the present inventors have found that by providing a first space in which an insert can be disposed and a second space communicating with the first space in a well, and having a configuration in which an upper end portion of a side surface defining the first space is formed in a C shape and the second space extends from a C-shaped opening portion in plan view, the second space can be used as a space into which a discharge tube such as a pipette can be inserted while stably holding the insert in the first space. Based on this novel finding, the present inventors have reached the following disclosure.

A cell culture substrate according to an aspect of the present disclosure is a cell culture substrate including at least one well including a side surface and a bottom surface, in which the side surface includes a first side portion defining a first space and a second side portion defining a second space communicating with the first space, and in plan view, an upper end portion of the first side portion is formed in a C shape having a central angle of 240 degrees or more, the second space extends from an opening portion of the upper end portion in a first direction away from the first space, and a facing surface of the second side portion, the facing surface facing the opening portion in the first direction, is disposed at a position where a maximum distance between the opening portion and the facing surface in the first direction is smaller than a diameter of the C shape of the upper end portion.

According to this configuration, the insert can be stably held in the first space using the first side portion including the upper end portion having a C-shape (that is, an arc shape having a central angle of 240 degrees) with a central angle of 240 degrees or more in plan view. For example, a commercially available general cylindrical insert can be held substantially evenly over its circumferential direction. Furthermore, the second space is a space extending from the opening portion of the C-shaped upper end portion of the first side portion in plan view, and even if a pipette or the like is inserted into the second space, the cells in the insert are hardly affected. Therefore, in a state where the insert is held in the first space of the well, it is possible to discharge and supply the culture medium using the second space while suppressing damage to cultured cells in the insert. Moreover, since the insert is stably held, the replacement of the culture medium in the insert can be easily performed.

Furthermore, since the maximum distance in the first direction between the opening portion of the upper end portion of the first side portion and the facing surface of the second side portion is smaller than the diameter of the upper end portion of the first side portion, it is possible to suppress an increase in the size of the well due to the provision of the second space. For this reason, for example, a plurality of wells can be arranged at a pitch similar to that of a conventional well plate.

When the culture medium is discharged, the bottom surface of the well may be tilted from a horizontal direction by tilting the cell culture substrate. As a result, since the culture medium in the first space can be easily moved to the second space by gravity, the culture medium can be efficiently discharged from the second space. Note that, since the insert is stably held in the first space, the position (relative position) of the insert with respect to the first side portion can be held even if the cell culture substrate is tilted. Therefore, it is possible to suppress the impact on the cells due to the contact of the insert with the first side surface. Furthermore, since a gap between the insert and the first side portion is partially narrowed, it is possible to prevent a part of the culture medium from remaining (liquid residue) without being discharged to that portion.

Moreover, when the cell culture substrate is used, TEER measurement can be easily performed. At the time of the TEER measurement, one of a pair of electrodes is inserted into the insert of the first space, and the other is inserted into the second space, so that the electrodes can be easily inserted. Furthermore, cell damage caused by contact between the electrodes and the insert can be suppressed.

In plan view, the facing surface of the second side portion may be smoothly curved so as to be convex in the first direction. With this configuration, liquid residue hardly occurs when the culture medium is discharged from the second space. Furthermore, since the second side portion has the above-described configuration, it is possible to insert an opening (for example, a distal end portion of a pipette) of a discharge tube for discharging the culture medium, and to define the second space having a reduced volume. Therefore, it is possible to further suppress an increase in the size of the well due to the provision of the second space.

The bottom surface may include a first bottom portion defining the first space and a second bottom portion defining the second space, and the first bottom portion and the second bottom portion may be flush with each other. With this configuration, the culture medium can be smoothly moved from the first space to the second space when the culture medium is discharged. Therefore, the culture medium can be discharged more efficiently. Furthermore, in a case where the cell culture substrate is tilted at the time of discharging the culture medium, a tilt angle can be suppressed to be small, so that the influence on the cultured cells by tilting the cell culture substrate can be reduced.

The bottom surface may include at least one groove, and the at least one groove may include a first groove continuous from a first portion in contact with the first side portion to a second portion in contact with the second side portion. With this configuration, the liquid located in the gap between the insert and the first side portion of the well is easily moved to a second space side by the groove. Therefore, the culture medium in the well can be discharged from the second space more efficiently.

Note that, when the gap between the first side portion and the insert is small, the insert can be more stably held in the first space, and liquid residue easily occurs in the gap due to capillary action. On the other hand, by providing the first groove on the bottom surface of the well, it is possible to reduce an amount of the culture medium remaining in the gap while suppressing the gap between the first side portion and the insert to be small. A width and a depth of the first groove may be designed such that a capillary force of the first groove can pull liquid from the gap between the first side portion and the insert.

The first side portion may be provided with a step portion so that the diameter of the upper end portion is larger than a diameter of a lower end portion in contact with the bottom surface, and the step portion may extend in a circumferential direction in plan view. With this configuration, in the gap between the insert and the first side portion of the well, it is possible to suppress the rise of a liquid level (an upper surface of the culture medium) by the step portion. Therefore, when the culture medium is discharged from the well, the old culture medium is less likely to remain in the gap. Furthermore, when the liquid level in the gap becomes too high, the liquid level in the second space S2 is lowered by a volume of the culture medium raised in the gap, and the liquid level in the insert may also be lowered (that is, the liquid amount is reduced) due to the siphon phenomenon. It is possible to suppress a decrease in the amount of the liquid (amount of the culture medium) in the insert due to the siphon phenomenon by suppressing an increase in the liquid level of the gap by the step portion.

The first side portion may include a support structure that supports a cell culture container (insert) in the first space at a distance from the bottom surface. As a result, the insert can be more stably held in the first space.

The cell culture substrate may include a plurality of wells. For example, in plan view, the at least one well may include a plurality of wells arranged in a row direction and a column direction intersecting the row direction. The first direction in each of the plurality of wells may be a direction intersecting both the row direction and the column direction. With this configuration, more wells can be arranged in a limited area. The row direction and the column direction may be orthogonal to each other. In this case, the plurality of wells may be arranged such that the first direction of each well forms an angle of, for example, 45 degrees with the row direction and the column direction in plan view.

The cell culture substrate may further include an outer wall surrounding the plurality of wells in plan view. The plurality of wells may include a plurality of outer wells located at ends in the row direction or the column direction, and a pool for accommodating water may be formed between a side wall forming the side surface of each of the plurality of outer wells and the outer wall. By filling the pool with water such as purified water, evaporation of the culture medium in the well can be suppressed. For example, the pool may be divided into more than or equal to a number of the plurality of outer wells. With this configuration, even when the cell culture substrate is tilted, the water stored in the pool is less likely to spill.

A cell culture method according to an aspect of the present disclosure is a cell culture method of, using the above-described cell culture substrate, and a cell culture container detachably accommodated in the first space of the at least one well while being separated from the bottom surface, culturing a cell in the cell culture container, the cell culture method including a discharge step of discharging a part or all of a culture medium held in the at least one well, in which the discharge step includes: a culture medium movement step of tilting the cell culture substrate in a state where the cell culture container is accommodated in the first space, and moving a culture medium located in the first space to the second space via the opening portion; and a step of inserting a discharge tube into the second space and discharging the culture medium located in the second space from the at least one well via the discharge tube. According to this method, it is possible to discharge the culture medium while suppressing an influence on the cultured cell. This method can be applied to a case where the culture medium is manually discharged from the well or a case where the culture medium is automatically discharged (for example, by a robot or the like). As a result of verification by the present inventors, the following was confirmed. When a conventional cell culture substrate is used, it is difficult to automatically perform the discharge step (that is, a step of tilting the cell culture substrate and discharging the culture medium). On the other hand, when the cell culture substrate of the present disclosure is used, since a pipette or the like can be inserted into the well while stably holding the insert, the discharge step can be automated. Furthermore, not only the culture medium in the well but also the culture medium in the insert can be automatically discharged.

Hereinafter, an embodiment of the present disclosure will be described with reference to the drawings. Note that the present disclosure is not limited by the embodiment. Furthermore, in the drawings, substantially the same members are denoted by the same reference signs.

Furthermore, in the following description, for convenience of explanation, terms indicating directions such as "upper", "lower", and "side" are used on the assumption of a state at the time of normal use, but it does not mean to limit the use state or the like of a cell culture substrate according to the present disclosure.

<<Embodiment>>

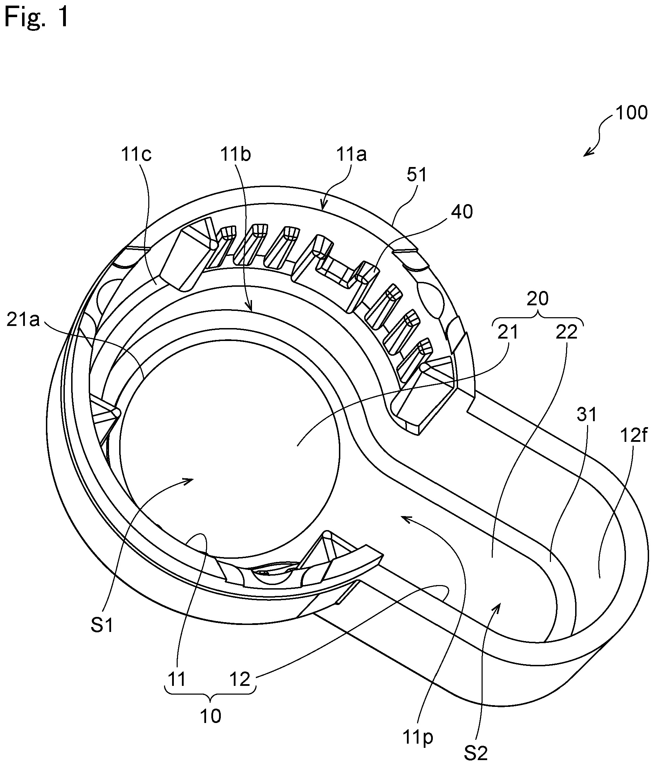

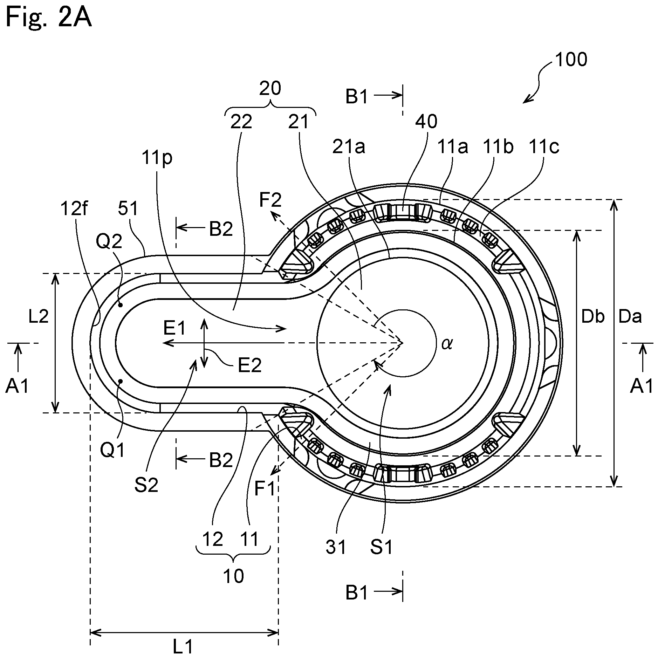

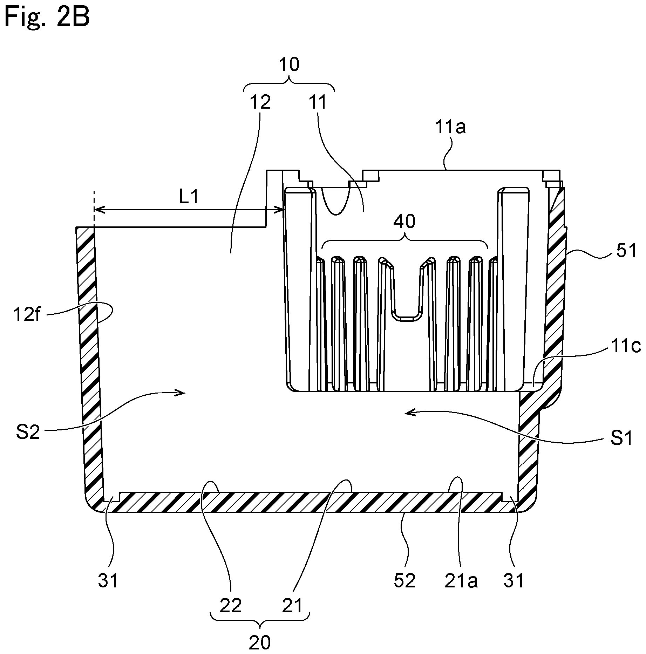

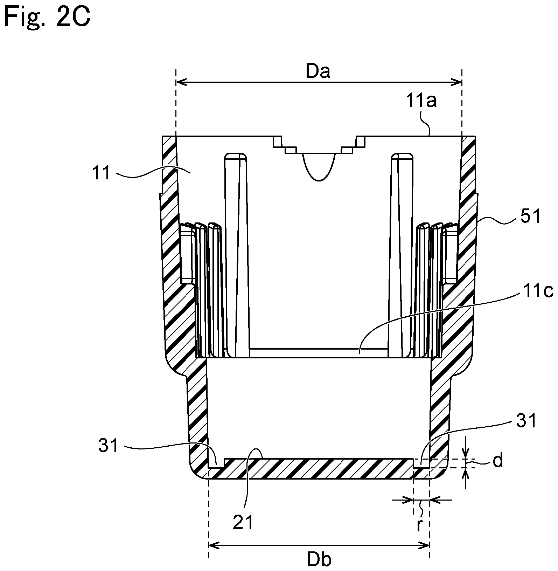

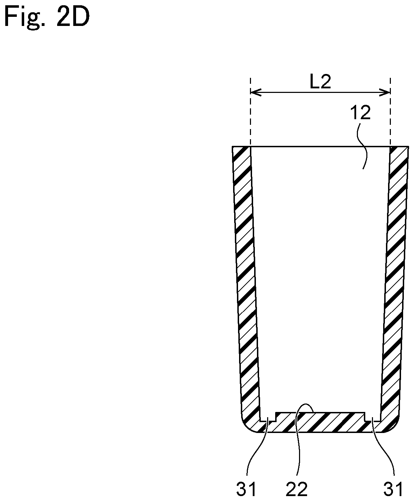

An overall configuration of a cell culture substrate according to an embodiment of the present disclosure will be described with reference to Fig. 1 and Figs. 2A to 2D. Fig. 1 is a perspective view of the cell culture substrate according to an embodiment of the present disclosure. Fig. 2A is a plan view of the cell culture substrate of Fig. 1. Fig. 2B is a cross-sectional view taken along line A1-A1 of Fig. 2A, Fig. 2C is a cross-sectional view taken along line B1-B1 of Fig. 2A, and Fig. 2D is a cross-sectional view taken along line B2-B2 of Fig. 2A.

As illustrated in Fig. 1 and Figs. 2A to 2D, the cell culture substrate according to the present embodiment includes at least one well 100 including a side surface 10 and a bottom surface 20, a side wall 51 constituting the side surface 10 of the well 100, and a bottom wall 52 constituting the bottom surface 20. The cell culture substrate is, for example, a resin molded body formed by injection molding.

The side surface 10 of the well 100 includes a first side portion 11 defining a first space S1 and a second side portion 12 defining a second space S2. The second space S2 communicates with the first space S1. The first space S1 is a space for culturing cells. The first space S1 is configured such that an insert (not illustrated) can be detachably disposed. The second space S2 is a space that can be used for supply, discharge, and the like of a culture medium. The second space S2 is configured such that a distal end portion of a pipette, an aspirator, or the like, an electrode for TEER measurement, or the like can be inserted without being in contact with the insert.

The first side portion 11 includes an upper end portion 11a and a lower end portion 11b in contact with the bottom surface 20. In plan view, the upper end portion 11a of the first side portion 11 is formed in a C shape (arc shape) having a central angle $\alpha$ of 240 degrees or more. The "plan view" refers to a state viewed from above the well 100 in a thickness direction of the bottom wall 52. With this configuration, the insert can be stably held in the first space S1. For example, by providing a plurality of support structures on the first side portion 11 or an upper surface of the side wall 51 constituting the first side portion 11, the insert can be held at a plurality of places. In the case of a general cylindrical insert, an upper end portion or an outer surface of the insert may be supported at three places equally divided into three in the circumferential direction. As the central angle $\alpha$ is larger, it is easier to stably hold the insert. The central angle $\alpha$ is, for example, 300 degrees. Note that, when the central angle $\alpha$ becomes too large, a width of an opening portion 11p becomes small, and there is a possibility that the liquid in the first space S1 hardly moves to the second space S2 through the opening portion 11p. The central angle $\alpha$ may be set such that the opening portion 11p has a width that allows liquid to easily pass therethrough.

In plan view, the second space S2 extends from the opening portion 11p of the upper end portion 11a in a first direction E1 away from the first space S1. In plan view, the second side portion 12 includes a facing surface 12f facing the opening portion 11p of the upper end portion 11a in the first direction E1. In plan view, the facing surface 12f of the second side portion 12 may be smoothly curved so as to be convex in the first direction E1. With this configuration, liquid residue hardly occurs when the culture medium is discharged from the second space S2. Furthermore, since the second side portion 12 includes the facing surface 12f as described above, a distal end portion of a pipette or the like for discharging a culture medium can be easily inserted, and the second space S2 having a reduced volume can be defined. In plan view, the facing surface 12f may have, for example, an arc shape (here, a semicircular shape). As illustrated in Fig. 2B, the facing surface 12f may be tilted such that a distance from the opening portion 11p increases toward the upper side.

A maximum distance (that is, a maximum length of the second space S2 in the first direction E1)L1 between the opening portion 11p and the facing surface 12f of the second side portion 12 in the first direction E1 is smaller than a diameter Da of the C shape of the upper end portion 11a. With this configuration, an increase in the size of the well 100 can be suppressed. Furthermore, the wells 100 can be arranged at a pitch according to the standard. The diameter Da of the arc of the upper end portion 11a is, for example, 16.0 mm, and the maximum distance L1 is, for example, 10.5 mm. In plan view, a maximum width L2 of the second space S2 in a second direction E2 orthogonal to the first direction E1 may be, for example, a width or less of the opening portion 11p in the second direction E2. The maximum width L2 of the second space S2 is, for example, 7.7 mm.

The first direction E1 may be a radial direction of the C-shaped upper end portion 11a. With this configuration, when the cell culture container is tilted to discharge the culture medium, the liquid located in the first space S1 can be easily moved to the second space S2, and an amount of the culture medium remaining in the first space S1 can be reduced. Furthermore, in the TEER measurement, a pair of electrodes can be easily inserted into the vicinity of the center of the insert disposed in the first space S1 and the second space S2, respectively. The planar shape of each well 100 may be line-symmetric with a line connecting a center of the upper end portion 11a and a center of the opening portion 11p as an axis.

The bottom surface 20 includes a first bottom portion 21 defining the first space S1 and a second bottom portion 22 defining the second space S2. The first bottom portion 21 includes an observation surface 21a for performing microscopic observation and the like. The observation surface 21a has, for example, a circular shape with a diameter of 9.5 mm. The observation surface 21a may be disposed at a distance from a peripheral edge of the first bottom portion 21. In a case where the insert is disposed, the observation surface 21a at least partially overlaps a bottom surface of the insert in plan view. The first bottom portion 21 and the second bottom portion 22 may be flush with each other. With this configuration, the liquid located in the first space S1 can be easily moved to the second space S2, so that the culture medium can be discharged more efficiently. An area of the bottom surface 20 in plan view is, for example, 187 mm$^2$, which is the same as a bottom area of a well in a commercially available well plate.

When the culture medium is discharged, for example, the cell culture substrate may be tilted such that the first bottom portion 21 of the well 100 is relatively lower than the second bottom portion 22. The cell culture substrate may be tilted, for example, in the first direction E1. A direction in which the cell culture substrate is tilted (tilt direction) is not limited to the first direction E1, and may be a direction having a component in the first direction E1, that is, a direction of less than $\pm$ 90 degrees with respect to the first direction E1. The tilt direction is preferably a direction forming an angle of $\pm$ 45 degrees or less with the first direction E1. As a result, the culture medium in the first space S1 can be easily moved to the second space S2 by gravity. In this state, a distal end portion such as a pipette may be disposed in the vicinity of the lowest point of the second bottom portion 22 to discharge the culture medium. In a case where the cell culture substrate is tilted in directions F1 and F2 forming an angle of $\pm$ 45 degrees with the first direction E1, points Q1 and Q2 of the second bottom portion 22 become the lowest points.

<Step portion>

The first side portion 11 may be provided with a step portion such that the diameter Da of the upper end portion 11a of the first side portion 11 is larger than a diameter Db of the lower end portion 11b of the first side portion 11. The diameter Da is, for example, 16.0 mm, and the diameter Db is, for example, 12.5 mm. The step portion includes an upward step surface 11c located between the upper end portion 11a and the lower end portion 11b. In plan view, the step portion extends in the circumferential direction. With this configuration, it is possible to suppress the rise of a liquid level in a gap between the insert and the first side portion 11. Therefore, it is possible to suppress a decrease in an amount of the liquid (amount of the culture medium) in the insert due to the siphon phenomenon. Furthermore, when the culture medium is discharged from the well 100, the old culture medium is less likely to remain in the gap, so that liquid residue can be suppressed. The step portion may extend over the first side portion 11. That is, the step portion may extend in the circumferential direction from one end portion to the other end portion of the C shape in plan view.

A height (level) of the step surface 11c depends on the amount of the culture medium supplied to the well 100, but may be set to be about the same as the height of the liquid level in the insert. As a result, the height of the liquid level in the gap between the insert and the first side portion 11 and the height of the liquid level in the insert and the second space S2 can be equalized, so that the decrease in the amount of the culture medium in the insert due to the siphon phenomenon hardly occurs. A distance between the observation surface 21a and the step surface 11c in the thickness direction of the bottom wall 52 is, for example, 5.6 mm.

A width of the step surface 11c (width of the upper end portion 11a in the radial direction) is set to a width that can suppress the rise of the liquid level in the gap using the Coanda effect. On the other hand, when the width of the step surface 11c is too large, the diameter Da increases with respect to the diameter of the insert, and it may be difficult to stably hold the insert. Therefore, the width of the step surface 11c may be set so as to be able to suppress an increase in the liquid level while suppressing an increase in the diameter Da. The width of the step surface 11c is, for example, 0.8 to 1.3 mm.

At least a part of the second side portion 12 defining the second space S2 may not be provided with a step surface having the same height as the step surface 11c of the first side portion 11. As a result, it is possible to absorb the shake of the liquid level caused by the step surface 11c. In the illustrated example, such a step portion is not provided over the entire second side portion 12.

<Groove>

The bottom surface 20 may be provided with one or a plurality of grooves including a first groove 31. The grooves are disposed in a region other than the observation surface 21a in the bottom surface 20. A bottom portion of each of the grooves is located below the observation surface 21a.

The first groove 31 may be continuous from a first portion in contact with the first side portion 11 to a second portion in contact with the second side portion 12. With this configuration, the liquid located in the gap between the insert, and the first side portion 11 and the first bottom portion 21 of the well 100 can be easily moved to a second space S2 side by the first groove 31. Therefore, by disposing a distal end portion of a pipette or the like close to the second portion of the first groove 31, the culture medium in the well 100 can be more efficiently discharged from the second space S2.

The first groove 31 may extend along the side surface 10 from the first portion to the second portion. In the illustrated example, the first groove 31 is continuous from a first portion located between the first side portion 11 and the observation surface 21a to a second portion in contact with the facing surface 12f of the second side portion 12. In plan view, the first groove 31 may extend over the entire peripheral edge of the bottom surface 20.

A region of the bottom surface 20 where no groove is formed may have the same height (level) as the observation surface 21a. For example, a region of the first bottom portion 21 where no groove is formed (including the observation surface 21a) and a region of the second bottom portion 22 where no groove is formed may be flush with each other. In the illustrated example, the first groove 31 is provided only on the peripheral edge of the bottom surface 20, and a portion of the bottom surface 20 located inside the first groove 31 is flush. With this configuration, liquid residue caused by the unevenness of the bottom surface 20 can be reduced.

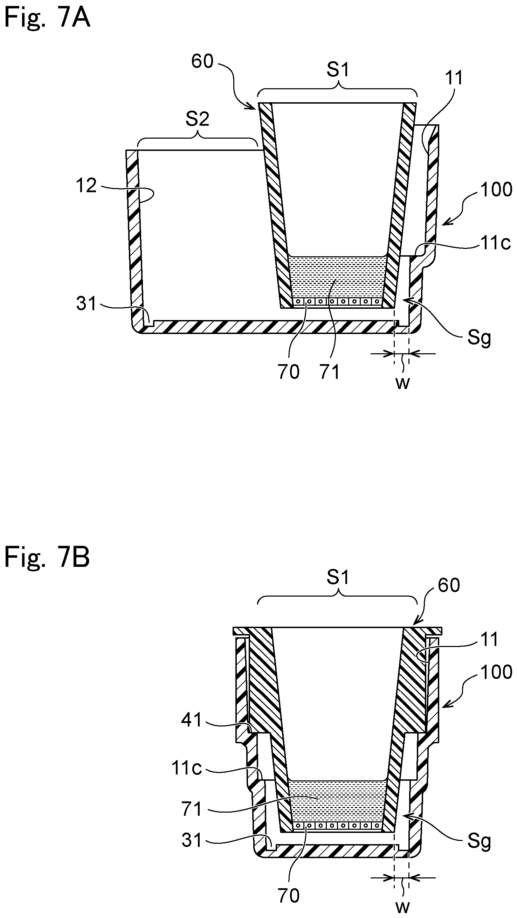

A width r of the first groove 31 may be smaller than a width w (see Fig. 7A and the like) of the gap between the insert and the first side portion 11 when the insert is supported at a predetermined position in the first space S1. By reducing the width r of the first groove 31, the culture medium remaining in the gap between the insert, and the first side portion 11 and the first bottom portion 21 can be pulled to the first groove 31 by capillary action and moved in the first groove 31 to the second space S2 side at the time of discharging the culture medium, so that the liquid residue can be further reduced. On the other hand, when the width of the first groove 31 is too small, the capillary force may be reduced. Therefore, the width r of the first groove 31 may be the minimum thickness or more of the side wall 51. Furthermore, from the viewpoint of securing the strength of a mold for forming the cell culture substrate, the width r of the first groove 31 may be, for example, 0.6 mm or more. The width r of the first groove 31 is, for example, 0.9 mm.

A depth (maximum depth) d of the first groove 31 may be, for example, the minimum thickness of or more the side wall 51. The depth d of the first groove 31 may be set such that the capillary force of the first groove 31 is higher than a capillary force generated in the gap between the insert, and the first side portion 11 and the first bottom portion 21. On the other hand, the depth d of the first groove 31 may be the maximum height H or less at which the liquid (here, the culture medium) rises in the gap between the insert and the first side portion 11. The depth d of the first groove 31 is, for example, 0.5 mm. Note that the "maximum height H" can be calculated from the width w of the gap between the insert and the first side portion 11, a surface tension $\sigma$ of the culture medium, a contact angle $\theta$ (for example, 90 degrees) of the culture medium with respect to a resin material of the insert and the well, a density $\rho$ of the culture medium, and gravitational acceleration g using the following Formula (1):

$$H = \frac{2\sigma(\cos\theta)}{\rho g w} \qquad (1)$$

The bottom surface 20 may be provided with another groove in addition to the first groove 31. The other groove may be connected to the first groove 31 or may be separated from the first groove 31. A width and a depth of the other groove may be the same as or different from those of the first groove 31.

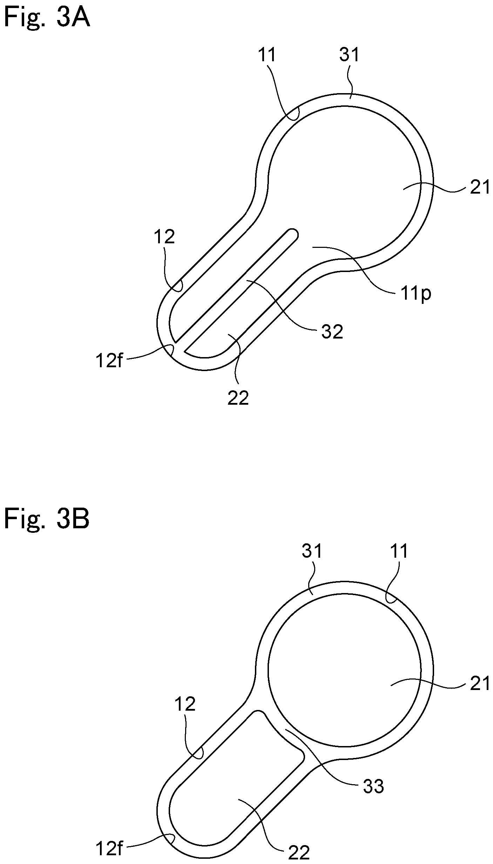

Figs. 3A to 3D are views illustrating variations in the shape and arrangement of the grooves, respectively, and are plan views illustrating the bottom surface 20 of the well 100. The peripheral edge of the bottom surface 20 is defined by lower end portions of the first side portion 11 and the second side portion 12.

As illustrated in Fig. 3A, the bottom surface 20 of the well 100 may be further provided with a second groove 32 extending from the vicinity of the opening portion 11p of the first side portion 11 to a portion of the first groove 31 in contact with the facing surface 12f of the second side portion 12. An end portion of the second groove 32 on a facing surface 12f side is connected to the first groove 31. As a result, liquid remaining in the gap between the insert, and the first side portion 11 and the first bottom portion 21 can be more efficiently guided to the facing surface 12f side via the second groove 32. Therefore, the time required for discharging the culture medium can be shortened.

As illustrated in Fig. 3B, a third groove 33 may be further provided in the opening portion 11p of the first side portion 11 of the bottom surface 20 of the well 100. In plan view, the third groove 33 is disposed so as to connect both ends of a portion of the first groove 31 formed in a C shape along the first side portion 11. Thus, the first groove 31 and the third groove 33 form an annular groove surrounding the bottom surface of the insert. Therefore, since the liquid remaining in the gap between the insert and the first side portion 11 and the first bottom portion 21 can be efficiently pulled out into the annular groove, the time required for discharging the culture medium can be shortened.

Note that, in a case where only the first groove 31 is provided, or in the example illustrated in Figs. 3A and 3B, most of the bottom surface of the well 100 is flat (flush with the observation surface). Therefore, it is possible to reduce the liquid residue caused by the unevenness of the bottom surface of the well 100 (unevenness caused by providing the grooves).

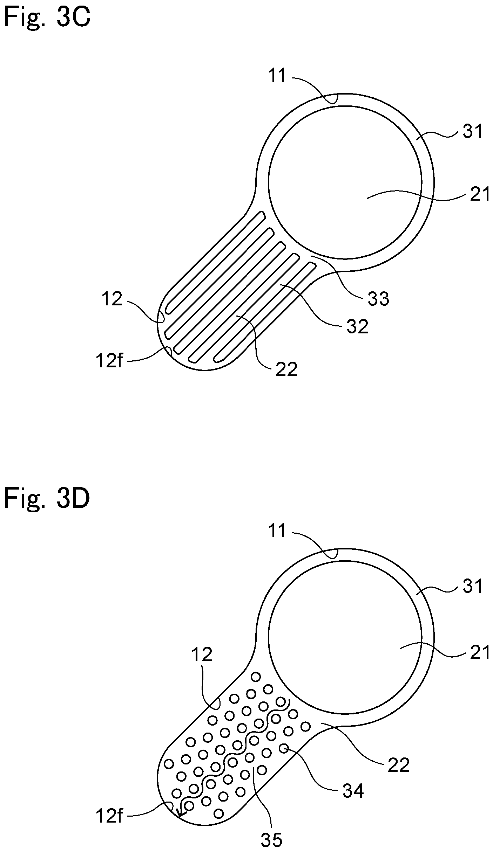

In the example illustrated in Fig. 3C, a plurality of the second grooves 32 is further provided in addition to the first groove 31 and the third groove 33 illustrated in Fig. 3B. In plan view, one end portion of each of the second grooves 32 is connected to the third groove 33, and the other end portion is connected to a portion of the first groove 31 in contact with the facing surface 12f of the second side portion 12. By providing the plurality of second grooves 32, the time required for discharging the culture medium can be shortened as compared with the examples illustrated in Figs. 3A and 3B.

As illustrated in Fig. 3D, a plurality of convex portions 34 may be provided on the second bottom portion 22. The plurality of convex portions 34 are arranged so as to define a groove that can be a flow path of the culture medium therebetween. A region 35 of the second bottom portion 22 where the plurality of convex portions 34 are not formed may be flush with the bottom portion of the first groove 31. A height (level) of an upper surface of each convex portion 34 may be the same as the height of the observation surface. The liquid remaining in the gap between the insert, and the first side portion 11 and the first bottom portion 21 can move the third groove 33 and the region 35 to a facing surface 12f side, so that the time required for discharging the culture medium can be shortened.

<Support structure>

Referring again to Figs. 1 and 2A to 2D. The first side portion 11 may include a support structure 40 for supporting the insert in the first space S1 at a distance from the bottom surface 20. Accordingly, the insert can be stably held in the first space S1. The insert is preferably disposed such that the center of the bottom surface of the insert substantially coincides with the center of the upper end portion 11a of the first side portion 11 in plan view. That is, it is preferable that the insert is not eccentrically disposed in the first space S1. Furthermore, the insert is preferably disposed such that the central axis of the insert is parallel to the thickness direction of the bottom wall 52 of the well 100 in a cross-sectional view. Accordingly, it is possible to prevent the gap between the first side portion 11 and the insert from being partially narrowed.

A part or all of the support structure 40 may be disposed on the step surface 11c or between the step surface 11c and the upper end portion 11a. As a result, the support structure 40 can be formed using a space enlarged in the radial direction by the step surface 11c. Furthermore, since the support structure 40 can be disposed above the liquid surface of the culture medium in the gap between the insert and the first side portion 11, it is possible to suppress an influence of the support structure 40 on the culture medium replacement work (for example, difficulty in discharging the culture medium).

The support structure 40 may be configured to be able to support one or a plurality of types of commercially available inserts. The support structure 40 includes a portion that supports a portion of the insert from below. The support structure 40 is preferably configured such that the insert can be attached and detached only by moving the insert in the vertical direction (in the thickness direction of the bottom wall 52) with respect to the support structure 40. Furthermore, the support structure 40 and a portion of the insert supported by the support structure 40 preferably have a dimensional relationship such that the insert can be easily attached to and detached from the well. As a result, it is possible to suppress damage to the cells due to attachment and detachment of the insert, for example, peeling of the cells seeded on the bottom surface of the insert.

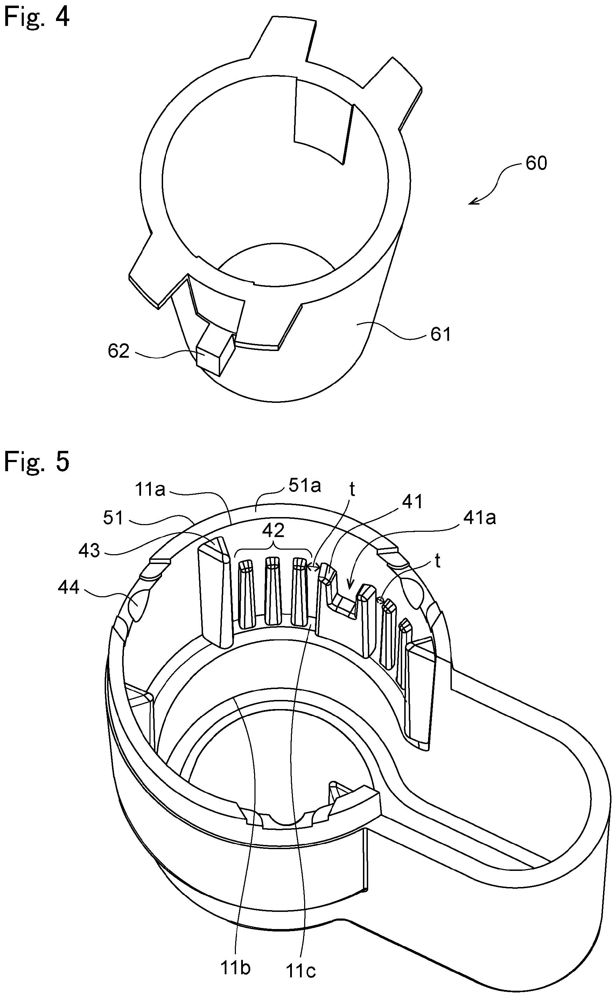

Fig. 4 is a perspective view illustrating an example of the insert. An insert 60 includes a cylindrical side wall and a bottom wall including a porous membrane filter. An outer surface 61 of the side wall of the insert 60 is provided with a plurality of (here, two) convex portions 62. The insert 60 is an insert manufactured by, for example, Falcon.

An example of the support structure 40 for supporting the insert 60 will be described with reference to a perspective view illustrated in Fig. 5.

The cell culture substrate includes, as the support structure 40, a plurality of ribs disposed between the upper end portion 11a and the lower end portion 11b of the first side portion 11. The plurality of ribs includes a plurality of support ribs 41, an incorrect insertion preventing rib 42 disposed between two adjacent support ribs among the plurality of support ribs 41, and a plurality of anti-rotation ribs 43. Lower surfaces of these ribs 41 to 43 are in contact with the step surface 11c. Since upper surfaces of the ribs 41 to 43 are located below the upper end portion 11a of the first side portion 11, it is possible to separately provide a support structure for supporting another type of insert at the upper end portion 11a of the first side portion 11. The ribs 41 to 43 may be positioned so as not to prevent insertion of the other type of insert.

Each of the plurality of (here, two) support ribs 41 has a recess 41a on the upper surface that receives one of the convex portions 62 in the insert 60. That is, a width of the recess 41a is larger than a width of each convex portion 62 of the insert 60.

The incorrect insertion preventing rib 42 is disposed at an interval t from each of the two adjacent support ribs 41. The interval t is smaller than a width of each convex portion 62 of the insert 60. The incorrect insertion preventing rib 42 may be divided into a plurality of portions. The plurality of divided portions may be arranged at intervals smaller than the width of each convex portion 62. By providing the incorrect insertion preventing rib 42, it is possible to prevent the insert 60 from being incorrectly inserted below a predetermined height. Therefore, it is possible to suppress the siphon phenomenon caused by the fact that the height of the liquid level in the insert 60 becomes lower than the height of the liquid level of the liquid (culture medium) filled in the well 100 due to erroneous insertion.

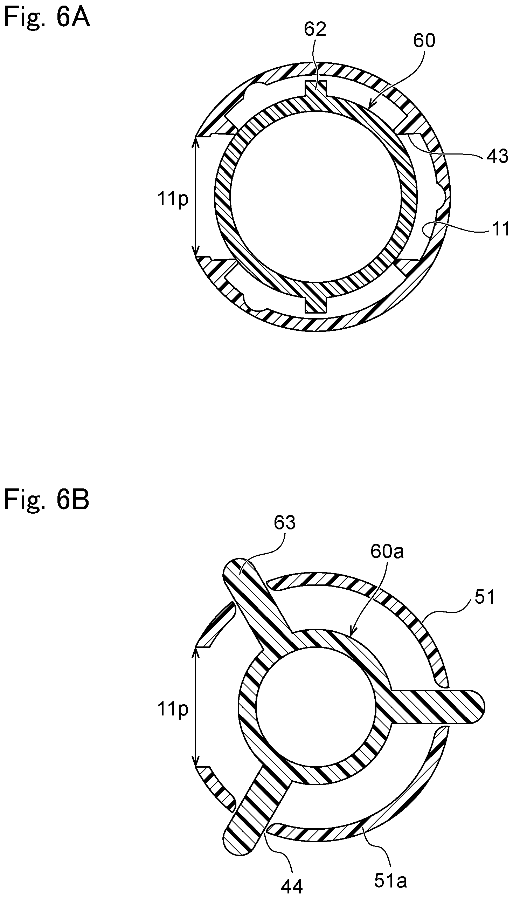

The plurality of (here, four) anti-rotation ribs 43 may be disposed at predetermined intervals in the circumferential direction of the first side portion 11. In plan view, each of the anti-rotation ribs 43 extends inward from the first side portion 11. A width of each anti-rotation rib 43 may decrease with distance from the first side portion 11. In the illustrated example, each anti-rotation rib 43 has a triangular prism shape. As illustrated in Fig. 6A, in plan view, the innermost portion of each anti-rotation rib 43 may be in contact with the outer surface of the insert 60. This can prevent the rotation of the insert 60.

The structure of the insert and the support structure 40 of the well 100 are not limited to the examples described above. A plurality of (here, three) cutout portions 44 may be formed on an upper surface 51a of the side wall 51 of the well 100. Thus, as illustrated in Fig. 6B, in a case where an insert 60a including a plurality of (here, three) protrusions 63 extending in a flange shape from one end portion of a cylindrical shape is used, each of the protrusions 63 of the insert 60a can be supported by each of the cutout portions 44. The insert 60a is, for example, an insert manufactured by FUJIFILM, manufactured by MILLIPORE, manufactured by VITRIGEL, or manufactured by GREINER. The three protrusions 63 of the insert 60a are disposed at positions equally dividing the upper end portion of the side wall of the insert 60a into three in the circumferential direction. Each cutout portion 44 is configured to receive one of the protrusions 63.

Furthermore, the upper surface 51a of the side wall 51 of the well 100 may not be provided with unevenness other than the cutout portion 44. Thus, for example, in a case where an insert (for example, manufactured by CORNING) including a flange at one end portion of a cylindrical shape is used, the flange of the insert can be supported by a flat region of the upper surface 51a of the side wall 51 other than the cutout portion 44.

In the example illustrated in Fig. 5, the support structure 40 is configured to be able to support a plurality of types of the inserts. Note that the support structure 40 may be configured to be able to support any one type of insert.

(Cell culture method)

An example of a method of culturing cells using the cell culture substrate of the present embodiment will be described. Figs. 7A to 12B are step cross-sectional views for explaining the cell culture method, respectively. Figs. 7A to 12A illustrate cross sections corresponding to line A1-A1 illustrated in Fig. 2A, and Figs. 7B to 12B illustrate cross sections corresponding to line B1-B1 illustrated in Fig. 2A.

As illustrated in Figs. 7A and 7B, the insert 60 is disposed in the first space S1 of the well 100 of the cell culture substrate, and cells 70 are seeded on the insert 60 by a known method. For example, a suspension containing the cells 70 and a culture medium 71 may be prepared and a predetermined amount of the suspension may be provided within the insert 60. The insert 60 is not particularly limited, and a known insert can be used. The insert includes a porous membrane filter on the bottom surface. The porous membrane filter is configured to permeate small molecules (chemical substances and the like) dissolved or suspended in a liquid, but not to permeate cells, for example. Here, the insert 60 illustrated in Fig. 4 is used as the insert 60.

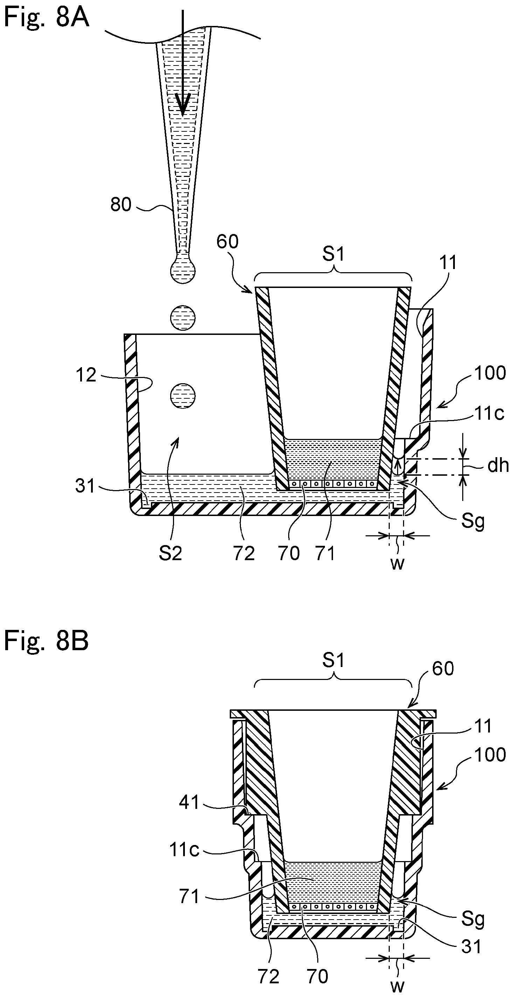

Then, as illustrated in Figs. 8A and 8B, a culture medium 72 is supplied into the well 100. Here, for example, the culture medium 72 is injected into the second space S2 of the well 100 using a pipette 80. At this time, when a width w of a gap Sg between the insert 60 and the first side portion 11 of the well 100 is small, a liquid level of the culture medium 72 in the gap Sg may rise due to capillary action. As a result, the liquid level of the culture medium 72 in the gap Sg becomes higher than a liquid level of a culture medium in the second space S2 by dh (> 0). In the present specification, the width w of the gap Sg refers to a distance between the lower end portion of the outer surface of the insert 60 and the first side portion 11 of the well 100 in the radial direction of the insert 60 in plan view. The width w of the gap Sg is, for example, about 1 to 2 mm.

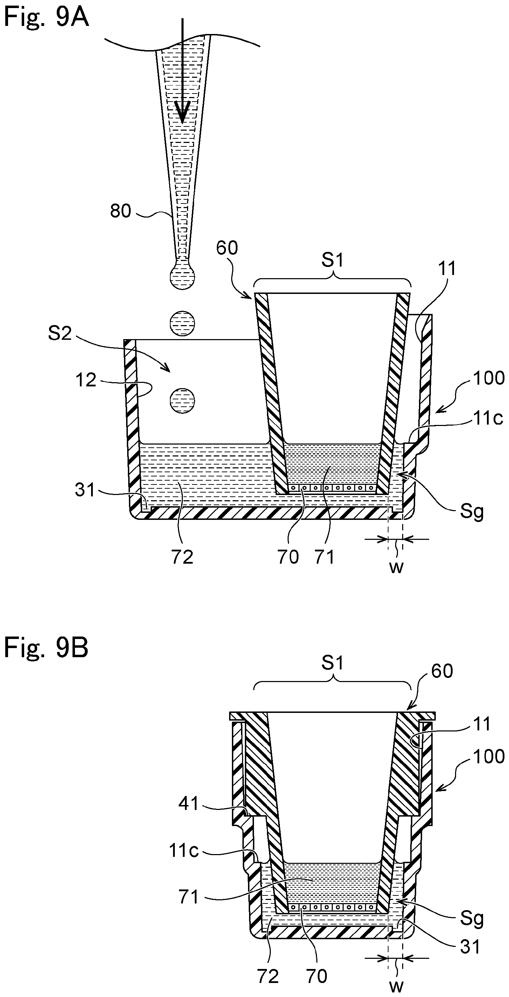

As illustrated in Figs. 9A and 9B, when the culture medium 72 is further injected into the well 100, and the liquid level of the culture medium 72 in the gap Sg reaches a height of the step surface 11c, the rise in the liquid level is suppressed by the Coanda effect. For example, the height of the liquid level in the gap Sg is substantially the same as the height of the step surface 11c. As a result, it is possible to suppress an increase in the difference dh of the liquid level height. By providing the step surface 11c in this manner, it is possible to suppress an increase in the liquid level in the gap Sg, and thus, it is possible to suppress a decrease in a liquid amount in the insert 60 due to the siphon phenomenon. Furthermore, by suppressing the rise of the liquid level in the gap Sg, liquid residue is less likely to occur in the gap Sg between the insert 60 and the first side portion 11 when the culture medium 72 is discharged.

Note that waves may be generated in the culture medium 72 due to an influence of the step surface 11c, and the liquid level may be shaken. In the present embodiment, since a step surface having the same height as the step surface 11c is not provided on the second side portion 12, the shake of the liquid level of the culture medium 72 due to the waves can be absorbed in the second space S2.

When the supply of the culture medium 72 is completed, an amount of the culture medium to be supplied to the insert 60 and the well 100 may be adjusted so that the height of the liquid surface of the culture medium 72 in the second space S2, the height of the liquid surface in the gap Sg regulated by the height of the step surface 11c, and the height of the liquid surface of the culture medium 71 in the insert 60 are substantially the same.

Thereafter, the well 100 is covered, and the cell culture substrate is allowed to stand in a $CO_2$ incubator humidified at a predetermined temperature (for example, 37°C) to culture the cells. The cells grow by taking necessary nutrients from the culture medium 72.

As the number of cells increases, the culture medium is replaced as necessary. The culture medium replacement includes a discharge step of discharging at least part of the old culture medium with reduced nutrients from the wells and a supply step of supplying the wells with a new culture medium after discharging the old culture medium.

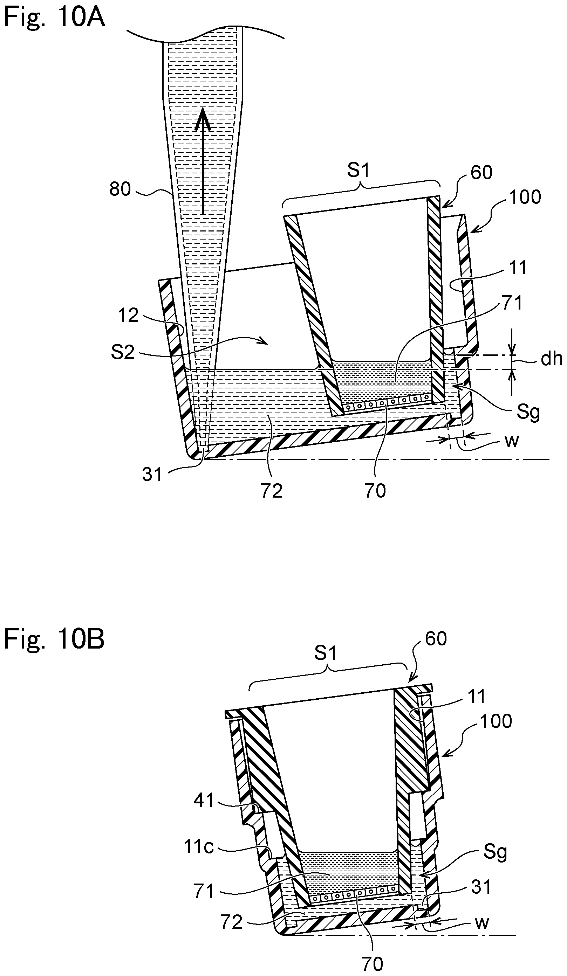

In the discharge step, as illustrated in Figs. 10A and 10B, first, the cell culture substrate is tilted while the insert is accommodated in the first space S1, and the second bottom portion of the second space S2 is made relatively lower than the first bottom portion of the first space S1. As a result, a part of the culture medium 72 located in the first space S1 is moved to the second space S2 through the opening portion of the first side portion 11 (culture medium movement step). Figs. 10A and 10B illustrate a state in which the cell culture substrate is tilted in the direction F2 illustrated in Fig. 2A. As illustrated, due to the capillary action, the liquid level in the gap Sg between the insert 60 and the first side portion 11 is located above the liquid level in the second space S2.

Next, the pipette 80 is inserted into the second space S2. A distal end portion of the pipette 80 may be disposed near the lowest point Q2 (see Fig. 2A) of the second bottom portion 22.

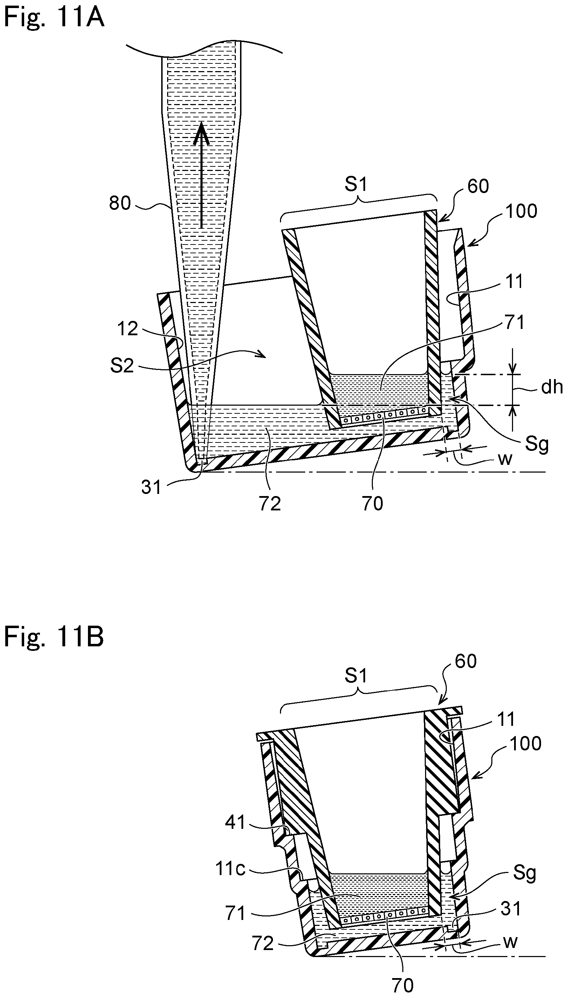

Subsequently, as illustrated in Figs. 11A and 11B, the culture medium 72 located in the second space S2 is discharged from the well 100 via the pipette 80. A portion of the culture medium 72 located in the gap Sg is more strongly affected by the capillary action than the gravity, and thus tends to remain in the gap Sg.

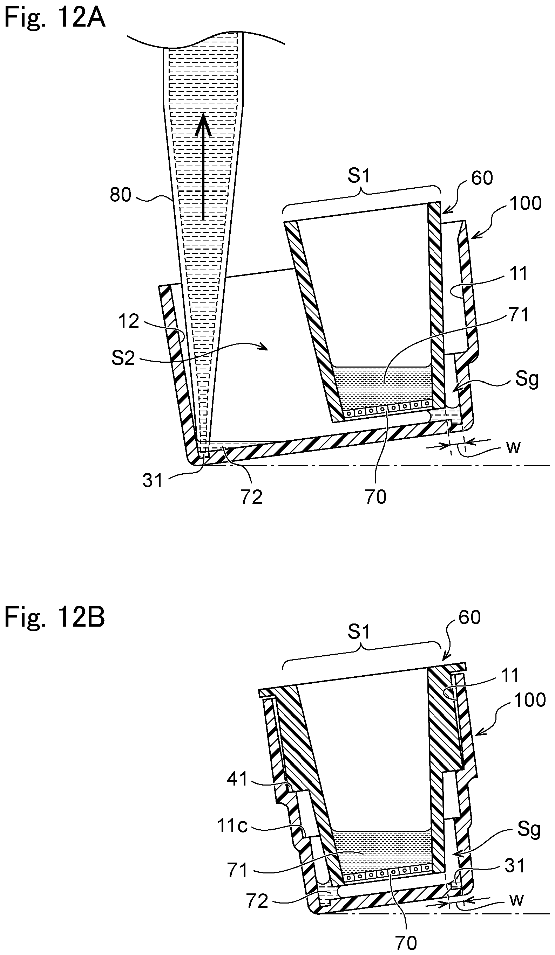

As illustrated in Figs. 12A and 12B, when most of the culture medium 72 in the second space S2 is discharged, the culture medium 72 remaining in the gap Sg is pulled by the capillary force of the first groove 31. The pulled culture medium 72 moves in the first groove 31 to the second space S2 side, and can be discharged from the well 100 by the pipette 80. In this way, by using the capillary force of the first groove 31, the amount of the old culture medium 72 remaining in the gap Sg can be reduced.

After discharging the old culture medium, a new culture medium is supplied into the well 100 (supply step). The method of supplying the new culture medium is similar to the method described above with reference to Figs. 8A to 9B.

In the above description, the cell culture method using the well 100 including the step portion and the first groove 31 has been described. However, even in a case where the well 100 does not include the step portion and/or the first groove 31, cells can be cultured in the same manner as described above. Even in a case where the step portion and the first groove 31 are not provided, the liquid residue can be reduced by decreasing a discharge speed of the culture medium or performing the discharge work of the culture medium a plurality of times.

(Another example of cell culture substrate)

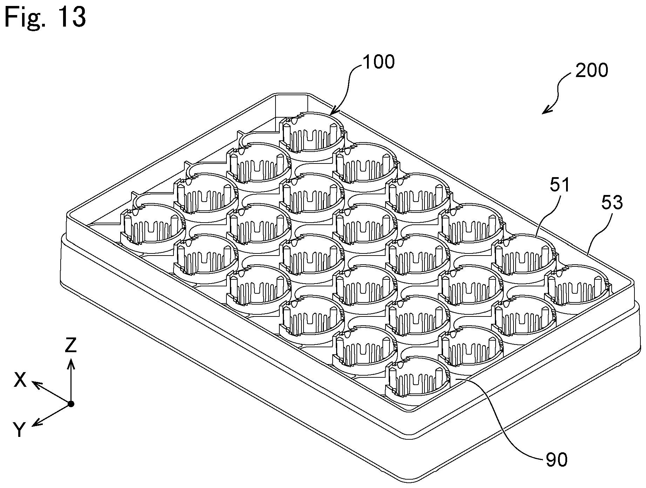

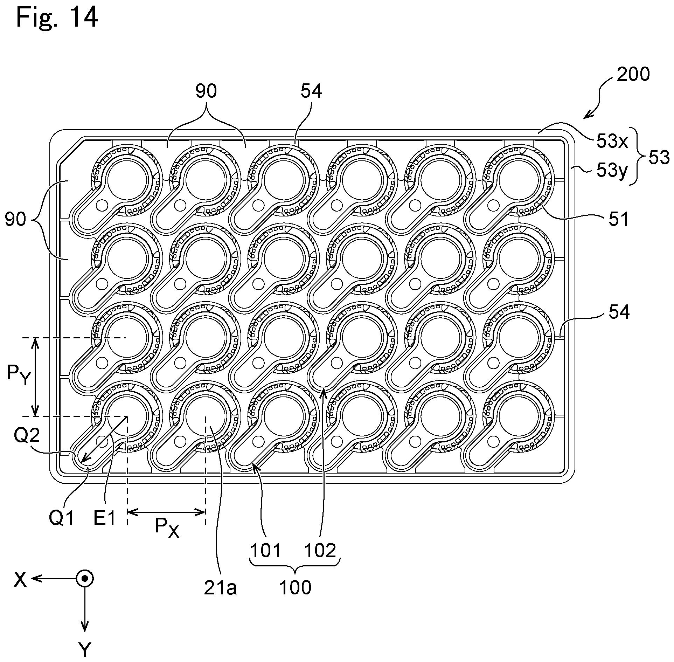

Fig. 13 is a perspective view illustrating an example of a cell culture substrate 200. Fig. 14 is a plan view of the cell culture substrate 200. The cell culture substrate 200 is a well plate including 24 wells 100 arranged two-dimensionally. In Figs. 13 and 14, the height direction of the cell culture substrate 200 is set to a Z direction. In plan view as viewed from the Z direction, the cell culture substrate 200 has, for example, a rectangular shape. Directions in which two sides (here, a long side and a short side) orthogonal to each other in the rectangle extend are defined as an X direction and a Y direction, respectively.

The cell culture substrate 200 includes a plurality of wells 100 and the outer wall 53 surrounding the plurality of wells 100 in plan view. The structure of each pf the wells 100 is similar to the structure described above with reference to Figs. 1 to 6B. In this example, the number of wells is 24, but is not limited to 24, and may be, for example, 6, 48, 96, or the like.

The outer wall 53 extends in a rectangular shape in plan view. In plan view, the outer wall 53 has two first outer wall portions 53x extending in the X direction and two second outer wall portions 53y extending in the Y direction. A length of each of the first outer wall portions 53x is, for example, 127.5 mm, and a length of each of the second outer wall portions 53y is, for example, 185.5 mm. A height of the outer wall 53 is, for example, 21.1 mm.

In plan view, the wells 100 are arranged, for example, in a row direction and a column direction intersecting (for example, orthogonal to) the row direction. Each well 100 is disposed such that the first direction E1 intersects both the row direction and the column direction that are arrangement directions of the wells 100. With this configuration, more wells 100 can be arranged in a limited area.

In the example illustrated in Fig. 14, the row direction of the well 100 is the X direction, and the column direction is the Y direction. The first direction E1 of each well 100 forms, for example, an angle of 45 degrees with each of the X direction and the Y direction. That is, the second space S2 of each well 100 extends in the X direction and the Y direction of the first space S1. The plurality of wells 100 is preferably arranged in the same direction (so that, for example, the first direction E1 forms an angle of 45° with the X direction and the Y direction). As a result, when the cell culture substrate 200 is tilted, the culture medium in the first space S1 of the plurality of wells 100 can be simultaneously moved to the second space S2.

An arrangement pitch Px of the wells 100 in the X direction and an arrangement pitch Py of the wells 100 in the Y direction may be the same. In this example, the arrangement pitches Px and Py are both 19 mm, which is the same as an arrangement pitch of commercially available well plates. As a result, an instrument such as a microscope or a pipette for observation corresponding to a commercially available well plate can also be applied to the cell culture substrate 200.

In the example illustrated in Fig. 14, the culture medium in each well 100 can be moved to the second space S2 by tilting the cell culture substrate 200 in the X direction, the Y direction, or both. "Tilting in the X direction" means that the two second outer wall portion 53y are tilted such that the one located on the second space S2 side of each well 100 is lower than the other. "Tilting in the Y direction" means that the two first outer wall portions 53x are tilted such that the one located on the second space S2 side of each well 100 is lower than the other. When tilted in the X direction, for example, a pipette or the like may be disposed in the vicinity of the lowest point Q2 of the second bottom portion 22 of each well 100 to suck the culture medium. When tilted in the Y direction, for example, a pipette or the like may be disposed in the vicinity of the lowest point Q1 of the second bottom portion 22 of each well 100 to suck the culture medium. Note that a tilt direction of the cell culture substrate 200 may be a direction forming an angle of ± 45 degrees or less with the first direction E1. Furthermore, the tilt direction of the cell culture substrate 200 may be changed stepwise at the time of discharging the culture medium.

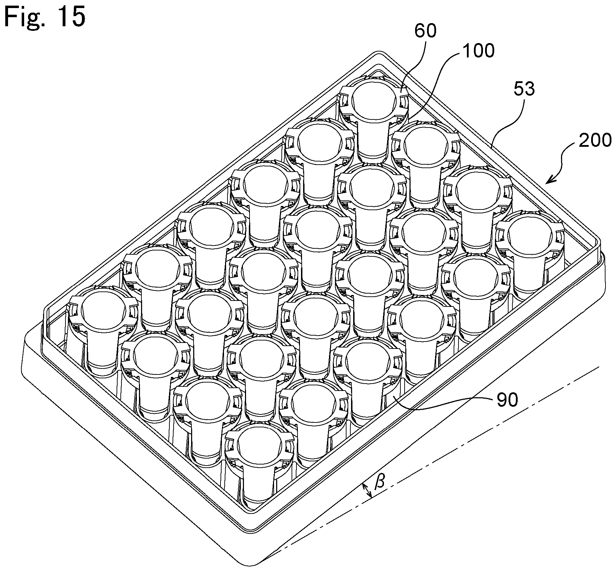

Fig. 15 is a perspective view illustrating a state in which the cell culture substrate 200 is tilted in the X direction. A tilt angle β of the bottom surface (the surface including the observation surface of the well) of each well 100 with respect to a horizontal plane may be larger than 0°. The tilt angle β is preferably greater than 5°. As a result, the moving speed of the culture medium into the second space S2 can be increased. On the other hand, the tilt angle β may be smaller than 20°. As a result, it is possible to reduce an influence on the cell by tilting or returning the cell culture substrate 200. The tilt angle β is, for example, about 10°. The work of tilting the cell culture substrate 200 may be performed manually or automatically.

Referring again to Figs. 13 and 14. The cell culture substrate 200 may further include a pool 90 between the outer wall 53 and the plurality of wells 100. By filling the pool 90 with water such as purified water, evaporation of the culture medium in the wells 100 can be suppressed.

The plurality of wells 100 include a plurality of outer wells 101 located at end portions in the row direction or the column direction and an inner well 102 located inside each of the outer wells 101. The pool 90 is disposed between the side wall 51 of each of the plurality of outer wells 101 and the outer wall 53. The pool 90 is divided into more than or equal to a number of the outer wells 101. By dividing the pool 90 into more than or equal to the number of the outer wells 101, the water stored in the pool 90 is less likely to spill even if the cell culture substrate 200 is tilted. In this example, the number of outer wells 101 is 16, and the pool 90 is divided into 19.

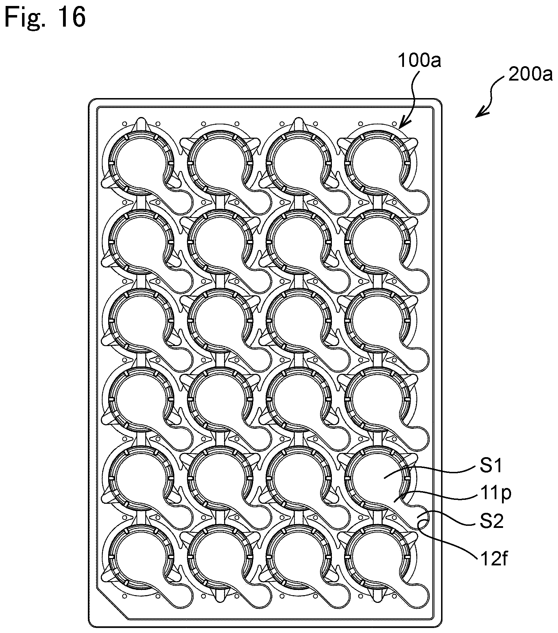

Fig. 16 is a plan view illustrating a modification of the cell culture substrate of the present embodiment. In a cell culture substrate 200a of the modification, the second space S2 includes a region having a width larger than that of the opening portion 11p of the C-shaped upper end portion of the first side portion in the vicinity of the facing surface 12f. The planar shape of each well 100 may not be line-symmetric.

In order to examine the usefulness of the cell culture substrate (well plate) 200 illustrated in Figs. 13 and 14, the present inventors conducted a cell culture experiment using the well plate 200, and confirmed the operability of the well plate 200 and evaluated the function of the cultured cells. Furthermore, the same experiment was performed using a commercially available well plate, and comparison with the case of using the well plate 200 was performed. Hereinafter, methods and results of cell culture experiments will be described.

<Cell culture experiment 1>

(Example 1)

In an example 1, the well plate 200 illustrated in Figs. 13 and 4 was prepared. An insert manufactured by Falcon was then disposed in each of the 24 wells in the well plate 200.

Subsequently, intestinal stem cells differentiated from hiPS cells were seeded in each insert, and differentiation induction into intestinal epithelial cells was performed under feederless conditions. The protocol of differentiation induction followed a method described in JP-A-2022-019411. For reference, the entire disclosure of JP-A-2022-019411 is incorporated herein by reference. If necessary, the culture medium was replaced by the following method, for example, at a frequency of once every 24 hours to 2 days. The culture experiment was terminated day 30 after cell seeding. After termination, the differentiated cells were evaluated by mRNA expression analysis and measurement of drug metabolizing enzyme activity.

Culture medium replacement method

Discharge and supply of the culture medium upon culture medium replacement was performed manually using the method described above with reference to Figs. 7A to 12B. Specifically, the well plate was tilted with the insert held in the first space of each well, and the culture medium was discharged from the second space of each well with a pipette. After discharge, the well plate was turned back horizontal to supply a fresh culture medium to the second space of each well.

Measurement of TEER value

From day 18 after cell seeding, a transepithelial electrical resistance (TEER) value of the cells was measured, and a temporal change in the TEER value was examined. For the measurement, a Millicell ERS-2 was used.

mRNA expression analysis

For the epithelial cells differentiated by the above method, expression levels of Villin1, which is an absorptive epithelial cell marker, MDR1 and PEPT1, which are drug transporters, and CYP3A4 and CYP2C9, which are drug metabolizing enzymes, were measured by an RT-qPCR method, and the relative expression level with respect to adult small intestine (ASI) was determined. For the measurement, KAPA SYBR Fast qPCR Kit was used.

Measurement of drug metabolizing enzyme activity

For the epithelial cells differentiated by the above method, metabolic activities of CYP3A4/5, CYP2C9, CYP2C19, and CYP2D6 were examined using midazolam, diclofenac, mephenytoin, and bufuralol as substrates. Here, the activities of CYP3A4/5, CYP2C9, CYP2C19, and CYP2D6 were calculated by measuring production amounts of 1'-hydroxylation of midazolam, 4'-hydroxylation of diclofenac, 4'-hydroxylation of mephenytoin, and 1'-hydroxylation of bufuralol.

(Example 2)

Differentiation induction into intestinal epithelial cells and evaluation of epithelial cells were performed in the same manner as in the example 1 except that an insert manufactured by Greiner was used as the insert.

(Comparative example 1)

Differentiation induction into intestinal epithelial cells and evaluation of epithelial cells were performed in the same manner as in the example 1 except that a commercially available well plate manufactured by Falcon having 24 wells and an insert manufactured by Falcon were used and the culture medium was replaced by the following method.

Culture medium replacement method

In the comparative example 1, the insert was removed from each well with tweezers, and the culture medium was discharged from the well using a pipette. Thereafter, the insert was returned into the well to supply the culture medium into the well. All these operations were performed manually.

(Comparative example 2)

Differentiation induction into intestinal epithelial cells and evaluation of epithelial cells were performed in the same manner as in the comparative example 1 except that a commercially available well plate manufactured by Greiner having 24 wells and an insert manufactured by Greiner were used.

(TEER value measurement results)

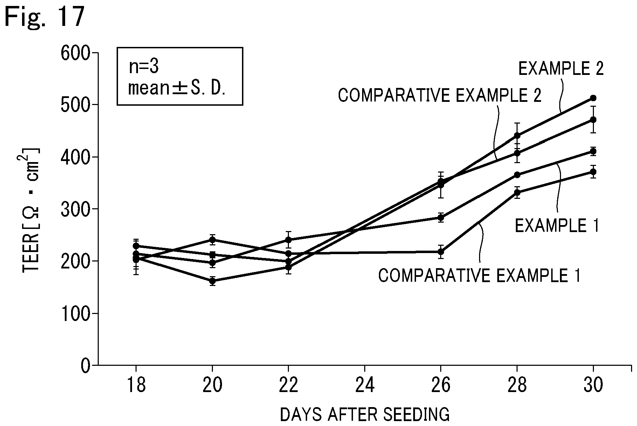

Fig. 17 is a diagram illustrating measurement results of TEER values from day 18 to day 30 after seeding in the examples 1 and 2 and the comparative examples 1 and 2. As illustrated in Fig. 17, TEER values of the cells of the examples 1 and 2 were TEER values or more of the cells of the comparative examples 1 and 2, respectively, and it was confirmed that even when the well plate 200 of the present embodiment was used, cells having a barrier function more than or equal to that in the case of using a commercially available well plate could be cultured.

Furthermore, particularly after day 24, the TEER values of the cells of the examples 1 and 2 were higher than the TEER values of the cells of the comparative examples 1 and 2, respectively, and it was found that the barrier function of epithelial cells was improved when the well plate 200 of the present embodiment was used. The reason for this is presumed as follows.

In the comparative examples 1 and 2, it is considered that since the insert was lifted up from the well with tweezers each time the culture medium was discharged, the cells in the insert were stimulated due to the vibration of the insert or the operation with the tweezers, and as a result of repeated stimulation, the barrier function was deteriorated. On the other hand, in the examples 1 and 2, since the culture medium in the well was discharged without removing the insert, it is presumed that an influence on the cells due to the replacement of the culture medium was suppressed, and the deterioration of the barrier function was suppressed. Furthermore, it is considered that since the insert was appropriately and stably held in each well, even if the well plate was tilted when the culture medium was discharged, the cells in the insert were hardly affected by the tilt of the well plate (even if affected, it was extremely small compared to the operation with tweezers). Moreover, in the examples 1 and 2, it is presumed that the culture medium replacement operation was smooth and the time required for the replacement was shortened, and the supply of the culture medium and the measurement of the TEER value were performed using the second space of the well, so that the influence of these operations on the cells was suppressed as compared with the comparative examples 1 and 2.

(Evaluation results)

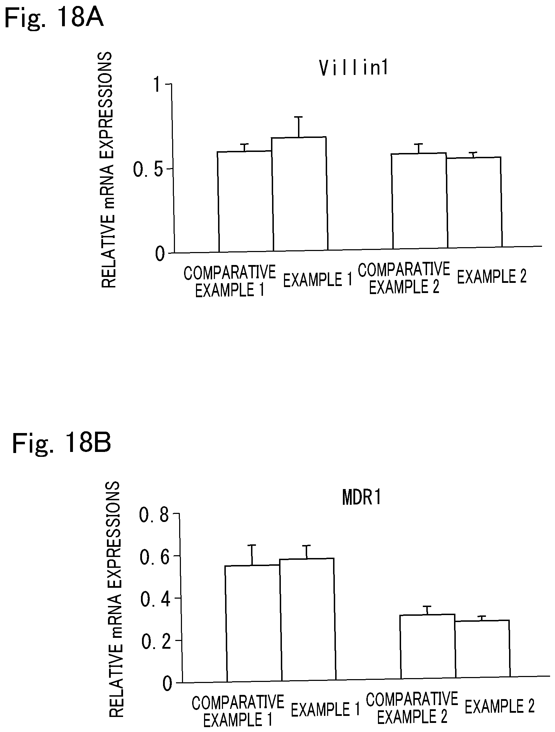

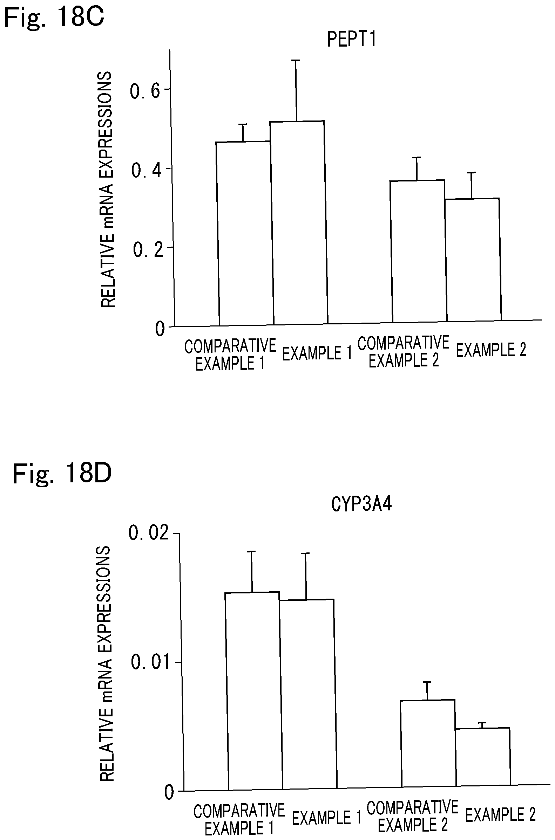

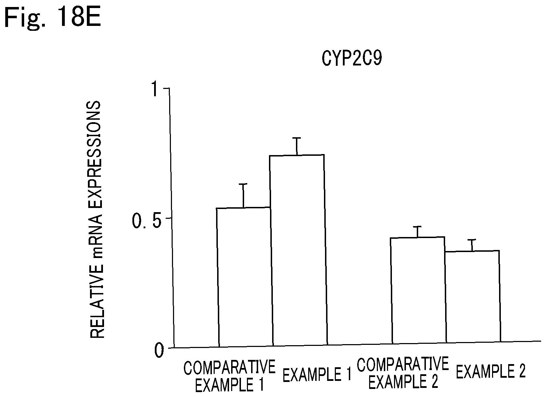

Figs. 18A to 18E are diagrams illustrating mRNA expression analysis results in epithelial cells differentiated in the examples 1 and 2 and the comparative examples 1 and 2. As illustrated in Figs. 18A to 18E, an mRNA expression level in the example 1 was comparable to that in the comparative example 1, and an mRNA expression level in the example 2 was comparable to that in the comparative example 2.

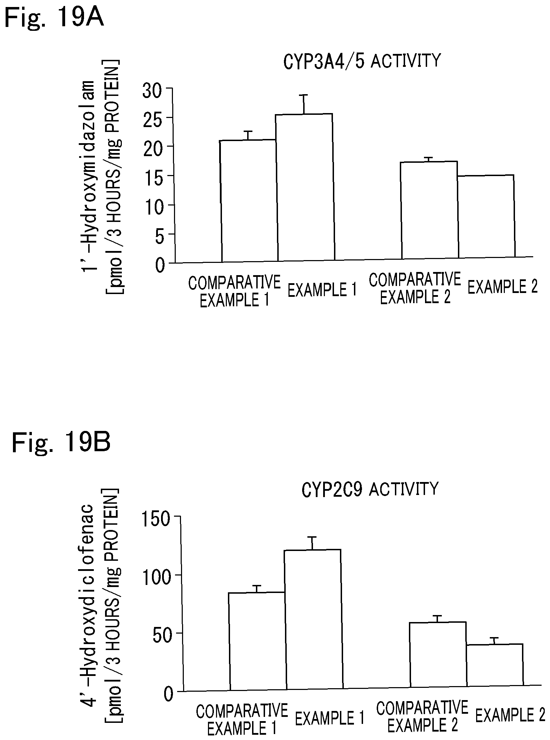

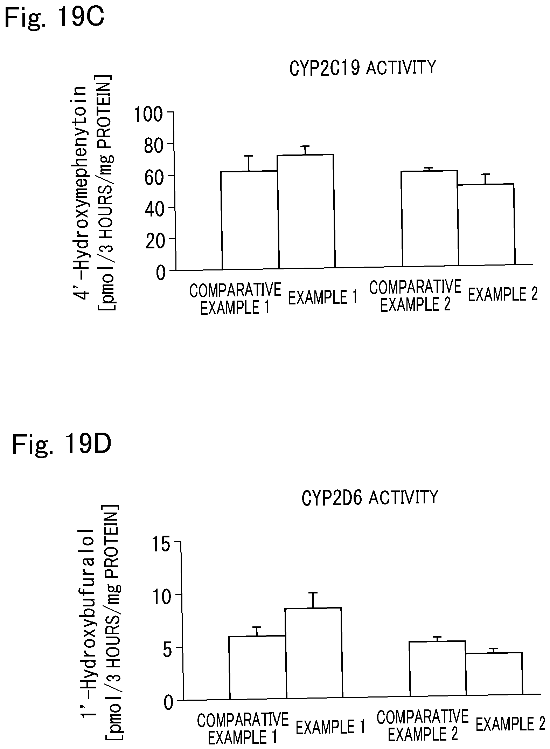

Figs. 19A to 19D are diagrams illustrating measurement results of the drug metabolizing enzyme activity in epithelial cells differentiated in the examples 1 and 2 and the comparative examples 1 and 2. As illustrated in Figs. 19A to 19D, the metabolic activity of each marker of the example 1 was comparable to that of the comparative example 1, and the metabolic activity of each marker of the example 2 was comparable to that of the comparative example 2.

From these evaluation results, it was confirmed that when the well plate 200 of the present embodiment is used, it is possible to culture cells having functions more than or equal to those in the case of using a commercially available well plate while greatly improving operability.

<Cell culture experiment 2>

(Example 3)

In an example 3, the cell culture substrate (well plate) 200 illustrated in Figs. 13 and 14 was prepared. Then, an insert manufactured by Greiner was disposed in each of the 24 wells in the well plate 200.

Subsequently, the three-dimensional intestinal organoids differentiated from hiPS cells were single-celled and seeded in each insert, and differentiation induction into intestinal two-dimensional organoids was performed under feederless conditions. The protocol of differentiation induction followed a method described in WO 2022/091020 A by the present inventors. The entire disclosure of WO 2022/091020 A is incorporated herein by reference. An amount of the culture medium on a basal side was 800 µL. If necessary, for example, the culture medium was replaced at a frequency of once every 24 hours to 3 days. Furthermore, a TEER value was measured from day 4 after seeding. The culture media replacement and the TEER value measurement were performed in the same manner as in the examples 1 and 2 described above. Moreover, the cell surface was observed with a phase contrast microscope to examine a change in the structure (villus-like structure) of the cell surface associated with differentiation. The culture experiment was terminated on day 10 after seeding. After termination, the differentiated cells were evaluated by the mRNA expression analysis.

mRNA expression analysis

For the intestinal organoids differentiated by the above method, expression amounts of LGR5 (intestinal stem cell marker), Villin1 (absorptive epithelial cell marker), MUC2 (goblet cell marker), Lysozyme (Paneth cell marker), Ki67 (proliferation cell marker), CYP3A4 (drug metabolizing enzyme), MDR1 (drug transporter), ALB (mature hepatocyte differentiation marker), OLFM4 (small intestine stem cell marker), CDX2 (transcription factor involved in proliferation and differentiation of intestinal epithelial cells) were measured by the RT-qPCR method. For the measurement, KAPA SYBR Fast qPCR Kit was used.

(Example 4)

Differentiation induction into intestinal two-dimensional organoids and evaluation of differentiated cells were performed in the same manner as in the example 3 except that an amount of the culture medium on the basal side was 1200 μL.

(Comparative example 3)

Differentiation induction into intestinal two-dimensional organoids and evaluation of differentiated cells were performed in the same manner as in the example 3 except for the use of a commercially available well plate manufactured by Greiner and an insert manufactured by Greiner having 24 wells, and the culture medium replacement method. In the comparative example 3, the culture medium was replaced in the same manner as in the comparative example 1.

(Cell observation results)

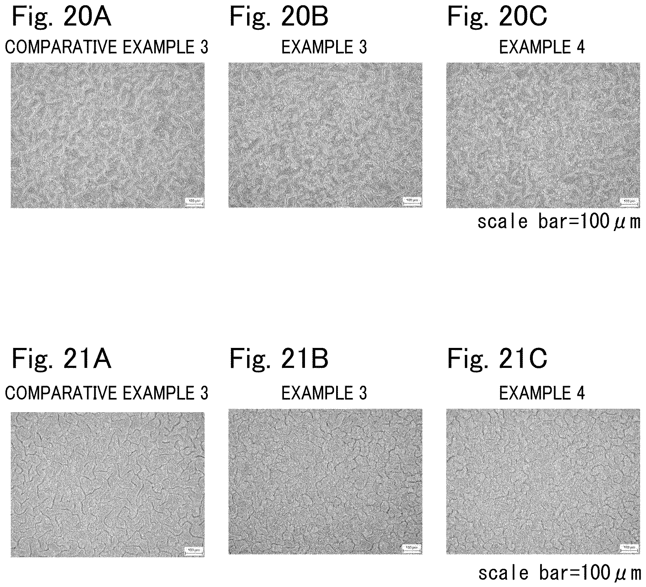

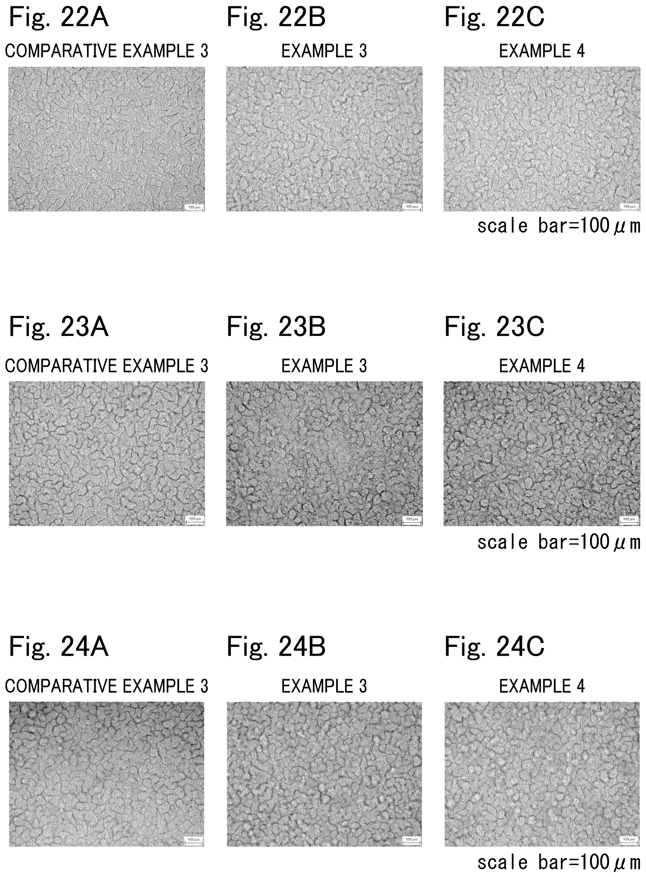

Figs. 20A to 20C are phase contrast microscopic images of the cell surface on day 6 after seeding in the comparative example 3, the example 3, and the example 4, respectively. Similarly, Figs. 21A to 21C to 24A to 24C are phase contrast microscopic images of the cell surface from day 7 to day 10 after seeding in the comparative example 3, the example 3, and the example 4, respectively.

From a villus density in the villus-like structure on the cell surface, it is possible to determine the degree of maturation of the cells. Comparing the results on the same day, it can be seen that the villus density of the cells of the examples 3 and 4 was higher than the villus density of the cells of the comparative example 3 (the villus was dense), and the maturation of the cells proceeded faster in the examples 3 and 4. Furthermore, for example, the villus density on day 10 after seeding in the comparative example 3 was comparable to the villus density on days 8 to 9 after seeding in the examples 3 and 4. From this, it can be seen that in the examples 3 and 4, the cells matured about 1 to 2 days earlier than in the comparative example 3.

Therefore, it was confirmed that the use of the well plate of the present embodiment promoted cell maturation as compared with the case of using a commercially available well plate. The reason why maturation was promoted is presumed as follows.

In the comparative example 3, it is considered that since the insert was lifted up from the well with tweezers each time the culture medium was discharged, the cells in the insert were stimulated due to the vibration of the insert or the operation with the tweezers, and the maturation of the cells was delayed. On the other hand, in the examples 3 and 4, it is presumed that since the culture medium in the well was discharged without removing the insert, the replacement of the culture medium was smooth and the time required for the replacement was shortened, and the supply of the culture medium and the measurement of the TEER value were performed using the second space of the well, the influence on the maturation rate of cells by each operation of the cell test was suppressed. Furthermore, it is considered that the insert was appropriately and stably held in each well, and the influence of tilting the well plate on the maturation rate of cells was hardly observed.

(TEER value measurement results)

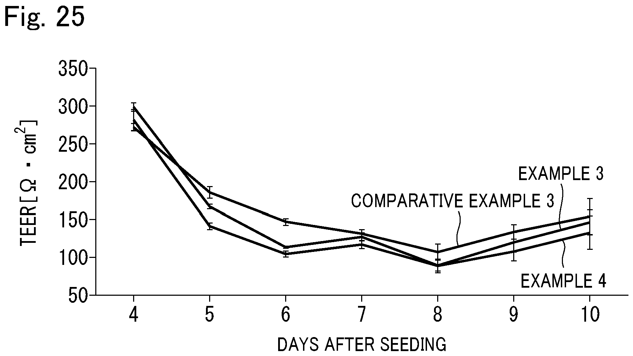

Fig. 25 is a diagram illustrating measurement results of TEER values from day 4 to day 10 after seeding in the examples 3 and 4 and the comparative example 3. As illustrated in Fig. 25, the TEER values of the cells of the examples 3 and 4 were comparable to the TEER values of the cells of the comparative example 3.

Note that specifically, in the examples 3 and 4, the TEER values were slightly lower than those in the comparative example 3, but this does not mean that the barrier function is deteriorated. In the intestinal two-dimensional organoids, it has been proved that the TEER value decreases when a crypt villus-like structure is formed, and since the TEER value is 100 $\Omega$cm$^{-2}$ or less in a living body, it is considered that in the examples 3 and 4, the intestinal two-dimensional organoids are rather in a state closer to the living body.

(Evaluation results)

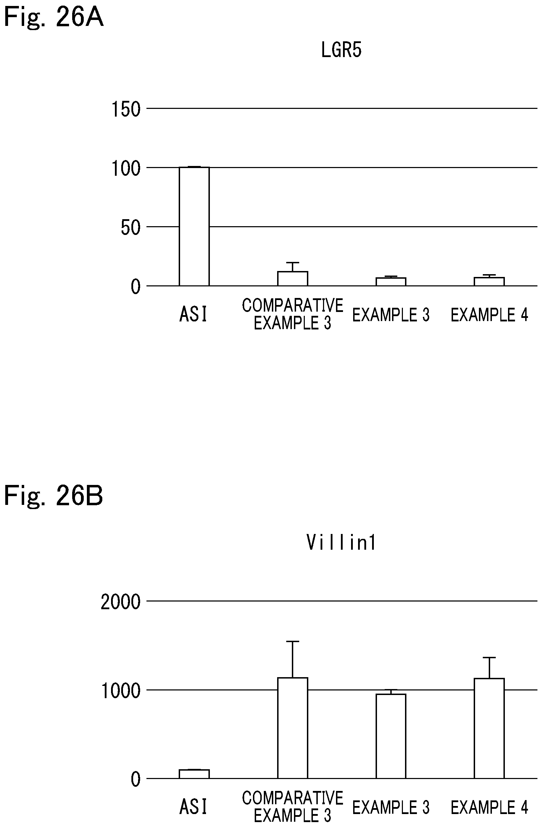

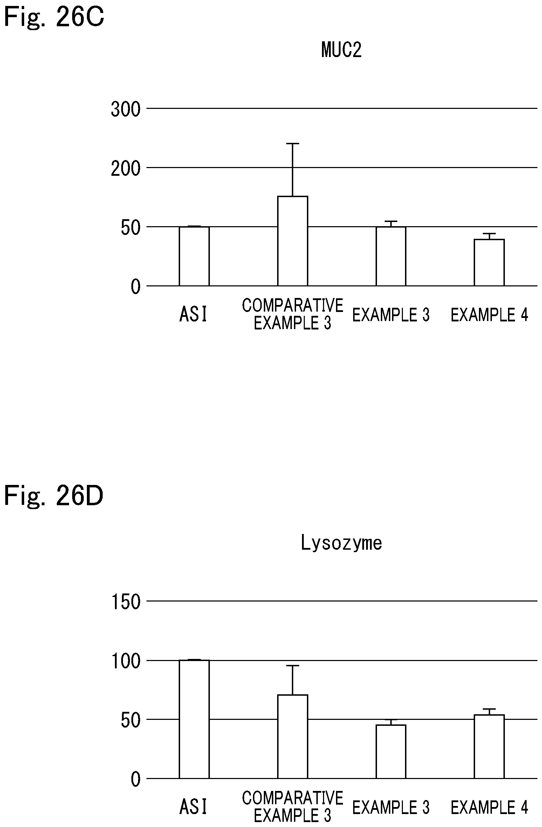

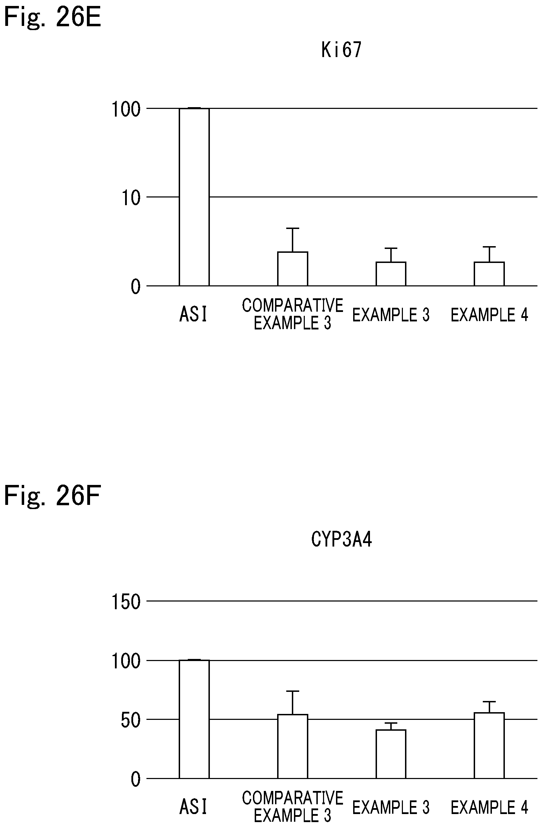

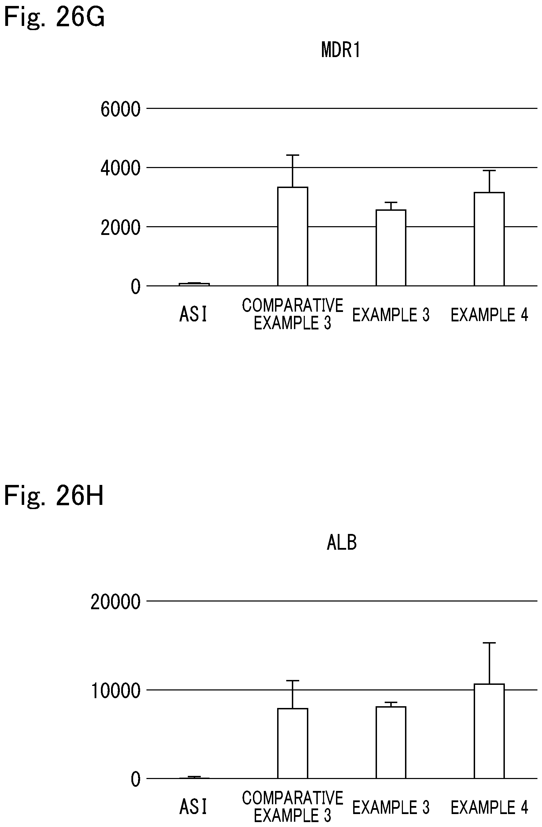

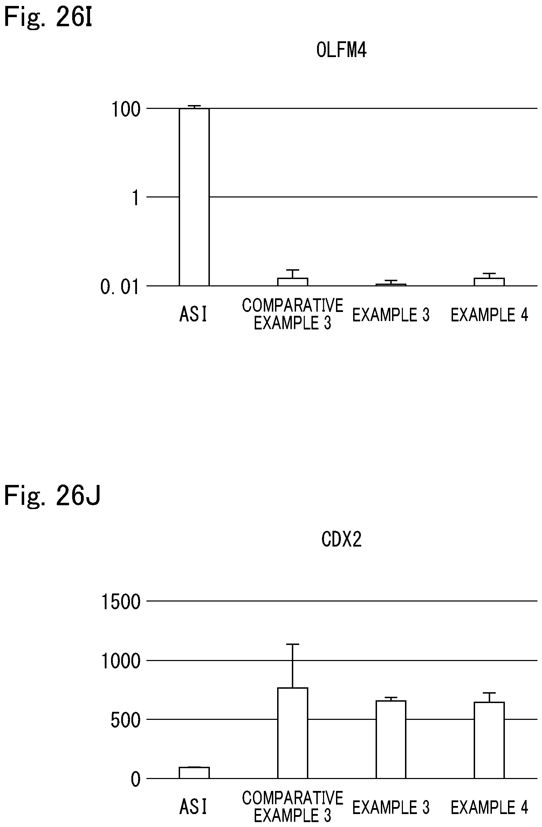

Figs. 26A to 26J are diagrams illustrating mRNA expression analysis results of markers for cells differentiated in the examples 3 and 4 and the comparative example 3. In each drawing, the expression level of ASI is also illustrated. As illustrated in Figs. 26A to 26J, mRNA expression levels in the examples 3 and 4 were comparable to those in the comparative example 3.

From the evaluation results of the cell culture experiment 2, it was confirmed that when the well plate 200 of the present embodiment is used, it is possible to mature cells at the same or higher rate using a commercially available well plate while greatly improving operability.

In the examples 1 to 4 described above, the replacement of the culture medium was performed manually, but the present inventors confirmed that the replacement of the culture medium can be automatically performed using an automated device by another cell culture experiment, and the operability can be further improved. The main factors that made the automation possible are that, when the well plate 200 of the present embodiment is used, the culture medium can be discharged from the well without removing the insert from the well, and the insert is appropriately held in the well even if the well plate is tilted. Moreover, the present inventors have also confirmed that even when the replacement of the culture medium is automated, the function of the cultured cells is more than or equal to that of the cells cultured using the conventional cell culture substrate.

A cell culture substrate and a cell culturing method according to the present disclosure can also be described as follows, for example.

[Item 1]

A cell culture substrate comprising at least one well including a side surface and a bottom surface, wherein

the side surface includes a first side portion defining a first space and a second side portion defining a second space communicating with the first space, and

in plan view, an upper end portion of the first side portion is formed in a C shape having a central angle of 240 degrees or more,

the second space extends from an opening portion of the upper end portion in a first direction away from the first space in plan view, and

the second side portion includes a facing surface facing the opening portion in the first direction, the facing surface being disposed at a position where a maximum distance between the opening portion and the facing surface in the first direction is smaller than a diameter of the C shape of the upper end portion in plan view.

[Item 2]

The cell culture substrate according to item 1, wherein the facing surface of the second side portion is smoothly curved so as to be convex in the first direction in plan view.

[Item 3]

The cell culture substrate according to item 1 or 2, wherein the bottom surface includes a first bottom portion defining the first space and a second bottom portion defining the second space, and the first bottom portion and the second bottom portion are flush with each other.

[Item 4]

The cell culture substrate according to any one of items 1 to 3, wherein

the bottom surface includes at least one groove, and the at least one groove includes a first groove continuous from a first portion in contact with the first side portion to a second portion in contact with the second side portion.

[Item 5]

The cell culture substrate according to any one of items 1 to 4, wherein the first side portion is provided with a step portion so that the diameter of the upper end portion is larger than a diameter of a lower end portion in contact with the bottom surface, and the step portion extends in a circumferential direction in plan view.

[Item 6]

The cell culture substrate according to any one of items 1 to 5, wherein the first side portion includes a support structure that supports a cell culture container in the first space at a distance from the bottom surface.

[Item 7]

The cell culture substrate according to any one of items 1 to 6, wherein

the at least one well includes a plurality of wells arranged in a row direction and a column direction intersecting the row direction in plan view, and the first direction in each of the plurality of wells is a direction intersecting both the row direction and the column direction in plan view.

[Item 8]

The cell culture substrate according to item 7, further comprising an outer wall surrounding the plurality of wells in plan view,

wherein the plurality of wells include a plurality of outer wells located at ends in the row direction or the column direction, and

a pool for accommodating water is formed between a side wall forming the side surface of each of the plurality of outer wells and the outer wall, and the pool is divided into more than or equal to a number of the plurality of outer wells.

[Item 9]

A cell culture method of culturing a cell in a cell culture container by using:

the cell culture substrate according to any one of items 1 to 8, and

the cell culture container detachably accommodated in the first space of the at least one well while being separated from the bottom surface, the cell culture method comprising:

a discharge step of discharging a part or all of a culture medium held in the at least one well, wherein

the discharge step includes:

a culture medium movement step of tilting the cell culture substrate in a state where the cell culture container is accommodated in the first space, and moving a culture medium located in the first space to the second space via the opening portion; and a step of inserting a discharge tube into the second space and discharging a culture medium located in the second space from the at least one well via the discharge tube.

Note that the present disclosure is not limited to the above-described embodiment, and can be implemented in various other modes. In the cell culture substrate including the plurality of wells, at least some of the wells may have the above-described structure. The cell culture substrate of the present disclosure may be applied to both open and closed systems. Furthermore, the present disclosure can be used not only in a case where an operation such as culture medium replacement is manually performed, but also in an automatic cell culture device that automatically performs the culture medium replacement.

INDUSTRIAL APPLICABILITY

Since the cell culture substrate according to the present disclosure can replace or discharge a culture medium while suppressing an influence on cultured cells, the cell culture substrate is useful for applications such as the pharmaceutical industry, the cosmetic industry, and the food industry.

REFERENCE SIGNS LIST

| 10 | side surface |
|---|---|
| 11 | first side portion |
| 11a | upper end portion of first side portion |
| 11b | lower end portion of first side portion |
| 11c | step surface |
| 11p | opening portion of upper end portion |
| 12 | second side portion |
| 12f | facing surface |
| 20 | bottom surface |
| 21 | first bottom portion |
| 21a | observation surface |
| 22 | second bottom portion |
| 31, 32, 33 | groove |
| 40 | support structure |
| 41 | support rib |
| 42 | incorrect insertion preventing rib |
| 43 | anti-rotation rib |
| 44 | cutout portion |
| 51 | side wall |
| 52 | bottom wall |
| 53 | outer wall |
| 60, 60a | insert |
| 70 | cell |
| 71, 72 | culture medium |
| 80 | pipette |
| 90 | pool |
| 100 | well |
| 101 | outer well |
| 200, 200a | cell culture substrate |
| Da | diameter of upper end portion of first side portion |
| E1 | first direction |
| E2 | second direction |
| Sg | gap between insert and first side portion |
| S1 | first space |
| S2 | second space |

**Claims**

1. A cell culture substrate comprising at least one well including a side surface and a bottom surface, wherein

the side surface includes a first side portion defining a first space and a second side portion defining a second space communicating with the first space, and
in plan view, an upper end portion of the first side portion is formed in a C shape having a central angle of 240 degrees or more,
the second space extends from an opening portion of the upper end portion in a first direction away from the first space in plan view, and
the second side portion includes a facing surface facing the opening portion in the first direction, the facing surface being disposed at a position where a maximum distance between the opening portion and the facing surface in the first direction is smaller than a diameter of the C shape of the upper end portion in plan view.

2. The cell culture substrate according to claim 1, wherein the facing surface of the second side portion is smoothly curved so as to be convex in the first direction in plan view.

3. The cell culture substrate according to claim 1 or 2, wherein the bottom surface includes a first bottom portion defining the first space and a second bottom portion defining the second space, and the first bottom portion and the second bottom portion are flush with each other.

4. The cell culture substrate according to claim 1 or 2, wherein

the bottom surface includes at least one groove, and

the at least one groove includes a first groove continuous from a first portion in contact with the first side portion to a second portion in contact with the second side portion.

5. The cell culture substrate according to claim 1 or 2, wherein the first side portion is provided with a step portion so that the diameter of the upper end portion is larger than a diameter of a lower end portion in contact with the bottom surface, and the step portion extends in a circumferential direction in plan view.

6. The cell culture substrate according to claim 1 or 2, wherein the first side portion includes a support structure that supports a cell culture container in the first space at a distance from the bottom surface.

7. The cell culture substrate according to claim 1 or 2, wherein

the at least one well includes a plurality of wells arranged in a row direction and a column direction intersecting the row direction in plan view, and

the first direction in each of the plurality of wells is a direction intersecting both the row direction and the column direction in plan view.

8. The cell culture substrate according to claim 7, further comprising an outer wall surrounding the plurality of wells in plan view,

wherein the plurality of wells include a plurality of outer wells located at ends in the row direction or the column direction, and

a pool for accommodating water is formed between a side wall forming the side surface of each of the plurality of outer wells and the outer wall, and the pool is divided into more than or equal to a number of the plurality of outer wells.

9. A cell culture method of culturing a cell in a cell culture container by using:

the cell culture substrate according to claim 1 or 2, and

the cell culture container detachably accommodated in the first space of the at least one well while being separated from the bottom surface, the cell culture method comprising:

a discharge step of discharging a part or all of a culture medium held in the at least one well,

wherein

the discharge step includes:

a culture medium movement step of tilting the cell culture substrate in a state where the cell culture container is accommodated in the first space, and moving a culture medium located in the first space to the second space via the opening portion; and

a step of inserting a discharge tube into the second space and discharging a culture medium located in the second space from the at least one well via the discharge tube.

Fig. 1

Fig. 2A

Fig. 2B

Fig. 2C

Fig. 2D

Fig. 3A

Fig. 3B

Fig. 3C

Fig. 3D

Fig. 4

Fig. 5

Fig. 6A

Fig. 6B

Fig. 7A

Fig. 7B

Fig. 8A

Fig. 8B

Fig. 9A

Fig. 9B

Fig. 10A

Fig. 10B

Fig. 11A

Fig. 11B

Fig. 12A

Fig. 12B

Fig. 13

Fig. 14

Fig. 15

Fig. 16

Fig. 17

Fig. 18A

Fig. 18B

Fig. 18C

PEPT1

Fig. 18D

CYP3A4

Fig. 18E

CYP2C9

Fig. 19A

CYP3A4/5 ACTIVITY

1'-Hydroxymidazolam [pmol/3 HOURS/mg PROTEIN]

Fig. 19B

CYP2C9 ACTIVITY

4'-Hydroxydiclofenac [pmol/3 HOURS/mg PROTEIN]

## Fig. 19C

CYP2C19 ACTIVITY

## Fig. 19D

CYP2D6 ACTIVITY

# Fig. 20A
## COMPARATIVE EXAMPLE 3

# Fig. 20B
## EXAMPLE 3

# Fig. 20C
## EXAMPLE 4

scale bar=100 $\mu$ m

# Fig. 21A
## COMPARATIVE EXAMPLE 3

# Fig. 21B
## EXAMPLE 3

# Fig. 21C
## EXAMPLE 4

scale bar=100 $\mu$ m

## Fig. 22A
COMPARATIVE EXAMPLE 3

## Fig. 22B
EXAMPLE 3

## Fig. 22C
EXAMPLE 4

scale bar=100μm

## Fig. 23A
COMPARATIVE EXAMPLE 3

## Fig. 23B
EXAMPLE 3

## Fig. 23C
EXAMPLE 4

scale bar=100μm

## Fig. 24A
COMPARATIVE EXAMPLE 3

## Fig. 24B
EXAMPLE 3

## Fig. 24C
EXAMPLE 4

scale bar=100μm

Fig. 25

Fig. 26A

LGR5

Fig. 26B

Villin1

Fig. 26C

MUC2

Fig. 26D

Lysozyme

Fig. 26E

Ki67

Fig. 26F

CYP3A4

Fig. 26G

MDR1

Fig. 26H

ALB

Fig. 26I

OLFM4

Fig. 26J

CDX2

## INTERNATIONAL SEARCH REPORT

| International application No. |
|---|
| **PCT/JP2023/018621** |

| **A.** | **CLASSIFICATION OF SUBJECT MATTER** |
|---|---|

*C12M 3/00*(2006.01)i; *C12N 5/071*(2010.01)i; *C12M 1/00*(2006.01)i
FI:  C12M3/00 Z; C12M1/00 A; C12N5/071

According to International Patent Classification (IPC) or to both national classification and IPC

| **B.** | **FIELDS SEARCHED** |
|---|---|

Minimum documentation searched (classification system followed by classification symbols)

C12M3/00; C12N5/071; C12M1/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| X | JP 2016-520307 A (CORNING INCORPORATED) 14 July 2016 (2016-07-14) | 1-3, 6, 7, 9 |
| | claims, paragraphs [0027], [0035], fig. 4, 5 | |
| Y | claims, paragraphs [0027], [0035], fig. 4, 5 | 8 |
| X | JP 5-041978 A (MILLIPORE CORP) 23 February 1993 (1993-02-23) | 1-3, 6, 7, 9 |
| | abstract, paragraph [0003], fig. 1, 8 | |
| Y | abstract, paragraph [0003], fig. 1, 8 | 8 |
| Y | WO 2019/202334 A1 (UNIVERSITY OF LEEDS) 24 October 2019 (2019-10-24) | 8 |
| | column 18, lines 11-19, fig. 4 | |
| A | JP 3208610 U (KANTO CHEM CO INC) 26 January 2017 (2017-01-26) | 1-8 |
| | claims, fig. 6 | |
| A | JP 8-332078 A (BECTON DICKINSON & CO) 17 December 1996 (1996-12-17) | 1-8 |
| | claims, fig. 1, 6, 7 | |

☐ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **27 July 2023** | **08 August 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/018621**

| Patent document cited in search report | | | Publication date (day/month/year) | Patent family member(s) | | | Publication date (day/month/year) |
|---|---|---|---|---|---|---|---|
| JP | 2016-520307 | A | 14 July 2016 | JP | 2019-162134 | A | |
| | | | | US | 2014/0322806 | A1 | |
| | | | | US | 2020/0392453 | A1 | |
| | | | | WO | 2014/179196 | A1 | |
| | | | | claims, paragraphs [0038], [0046], fig. 4, 5 | | | |
| | | | | EP | 3633022 | A1 | |
| | | | | US | 2018/0002660 | A1 | |
| JP | 5-041978 | A | 23 February 1993 | US | 5141718 | A | |
| | | | | abstract, column 1, lines 47-55, fig. 1, 8 | | | |
| | | | | EP | 483620 | A2 | |
| WO | 2019/202334 | A1 | 24 October 2019 | US | 2021/0147773 | A1 | |
| JP | 3208610 | U | 26 January 2017 | (Family: none) | | | |
| JP | 8-332078 | A | 17 December 1996 | US | 5534227 | A | |
| | | | | claims, fig. 1, 6, 7 | | | |
| | | | | EP | 747476 | A2 | |

Form PCT/ISA/210 (patent family annex) (January 2015)

**EP 4 527 917 A1**

**REFERENCES CITED IN THE DESCRIPTION**

**Patent documents cited in the description**

- WO 2018105078 A
- JP 2022019411 A
- WO 2022091020 A

54